(19) Europäisches Patentamt
European Patent Office
Office européen des brevets

(11) EP 3 536 399 A1

(12) # EUROPEAN PATENT APPLICATION
published in accordance with Art. 153(4) EPC

(43) Date of publication:
**11.09.2019 Bulletin 2019/37**

(21) Application number: **17885948.4**

(22) Date of filing: **22.12.2017**

(51) Int Cl.:
***B01J 13/00*** *(2006.01)* ***A61K 8/04*** *(2006.01)*
***A61K 8/63*** *(2006.01)* ***A61K 8/68*** *(2006.01)*

(86) International application number:
**PCT/JP2017/046172**

(87) International publication number:
**WO 2018/123883 (05.07.2018 Gazette 2018/27)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD TN**

(30) Priority: **27.12.2016 JP 2016253790**

(71) Applicant: **Kao Corporation**
**Chuo-Ku**
**Tokyo 103-8210 (JP)**

(72) Inventors:
- **NAITO Takaaki**
  **Wakayama-shi**
  **Wakayama 640-8580 (JP)**
- **KUMITA Yasukazu**
  **Wakayama-shi**
  **Wakayama 640-8580 (JP)**
- **ONISHI Yuka**
  **Wakayama-shi**
  **Wakayama 640-8580 (JP)**
- **FUKUDA Kimikazu**
  **Wakayama-shi**
  **Wakayama 640-8580 (JP)**
- **TAKAGI Michiya**
  **Wakayama-shi**
  **Wakayama 640-8580 (JP)**

(74) Representative: **Hoffmann Eitle**
**Patent- und Rechtsanwälte PartmbB**
**Arabellastraße 30**
**81925 München (DE)**

(54) # METHOD FOR PRODUCING CERAMIDE MICROPARTICLE DISPERSION

(57) A method for producing a ceramide microparticle dispersion causes a first mixture in a liquid state containing a ceramide and sterols and a second mixture in a liquid state containing water to separately flow, merges the first and second mixtures together, and passes a fluid formed by merging the first and second mixtures together through a small hole having a hole diameter of 0.03 mm or larger and 20 mm or smaller. A mass ratio of a content of a water-soluble organic solvent to a content of the ceramide in the first mixture is 0 or more and 30 or less.

FIG.2A

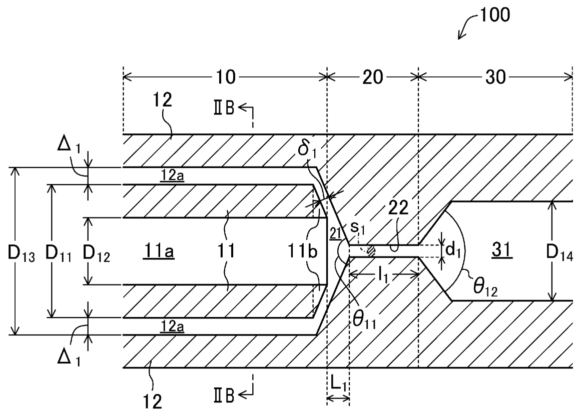




Printed by Jouve, 75001 PARIS (FR)

## Description

TECHNICAL FIELD

The present invention relates to a ceramide microparticle dispersion and a method for producing the same.

BACKGROUND ART

It has been known to add, to cosmetics, a ceramide as a moisturizing component imparting moisture to the skin. Some such cosmetics are prepared using a ceramide microparticle dispersion. Patent Documents 1 and 2 disclose, as a method for producing the ceramide microparticle dispersion, a method that causes an oil-based component containing a ceramide and a water-based component to flow separately, merges them together, and then passes a fluid formed by the merging through a small hole, for example. Patent Document 3 discloses a method that adds an oil-based component containing a ceramide and cholesterol to a water-based component, mixes them together using an ultrasonic homogenizer to prepare a preliminary dispersion, and further performs high-pressure emulsification treatment on the preliminary dispersion.

Patent Document 4 discloses a method for producing a ceramide dispersion composition excellent in dispersion stability. This method prepares an oil phase containing 1.35 g of a ceramide and 0.5 g of phytosterol dissolved in 80 g of ethanol as a water-soluble organic solvent, and mixes the oil phase and water having a volume seven times the volume of the oil phase together using a micromixer. Patent Document 5 discloses a method that prepares an oil phase containing 2.0 parts of a ceramide, 2.5 parts of a polyglycerin fatty acid ester, and 2.0 parts of cholesterol dissolved in 76.0 parts of ethanol as a water-soluble organic solvent, and mixes the oil phase and water having a mass seven times the mass of the oil phase together using a micromixer.

Patent Document 6 discloses a method for producing a liposome composition containing liposomes enclosing therein a ceramide that has been made amorphous. This method adds the ceramide, a dextrin fatty acid ester, phospholipid, cholesterol, and dipropylene glycol to purified water, mixes them together with stirring, and performs high-pressure treatment thereon.

CITATION LIST

PATENT DOCUMENT

Patent Document 1: Japanese Unexamined Patent Publication No. 2008-37842
Patent Document 2: Japanese Unexamined Patent Publication No. 2015-24404
Patent Document 3: Japanese Unexamined Patent Publication No. 2015-174840
Patent Document 4: Japanese Unexamined Patent Publication No. 2013-224314
Patent Document 5: International Publication No. WO 2009/145299
Patent Document 6: Japanese Unexamined Patent Publication No. 2014-208626

SUMMARY OF THE INVENTION

The present invention is directed to a method for producing a ceramide microparticle dispersion. The method includes the steps of: merging a first mixture in a liquid state containing a ceramide and sterols and a second mixture in a liquid state containing water together, the first and second mixtures having been caused to flow separately from each other; and passing a fluid formed by merging the first and second mixtures together at the step of merging through a small hole having a hole diameter of 0.03 mm or larger and 20 mm or smaller. A mass ratio of a content of a water-soluble organic solvent to a content of the ceramide in the first mixture is 0 or more and 30 or less.

The present invention is directed to a ceramide microparticle dispersion containing ceramide microparticles. A heat quantity of fusion of the ceramide microparticles in a temperature range of 45°C or higher and 80°C or lower is 0.30 J/g or less.

BRIEF DESCRIPTION OF THE DRAWINGS

FIG. 1 is a diagram of a configuration of a micromixer system.

FIG. 2A is a vertical cross-sectional view of a portion of a principal part of a micromixer of a first configuration.
FIG. 2B is a cross-sectional view taken along line IIB-IIB in FIG. 2A.
FIG. 3 is a vertical cross-sectional view of a portion a principal part of a micromixer of a second configuration.
FIG. 4A is a graph of a relation between a cholesterol content and an average particle diameter of ceramide microparticles in ceramide microparticle dispersions.
FIG. 4B is a graph of a relation between a cholesterol content and a heat quantity of fusion of ceramide microparticles in ceramide microparticle dispersions.
FIG. 5A is a graph of a relation between a temperature of a second mixture and an average particle diameter of ceramide microparticles.
FIG. 5B is graph of a relation between a temperature of the second mixture and a heat quantity of fusion of ceramide microparticles.
FIG. 6A is a graph of a relation between a hole diameter of a small hole and an average particle diameter of ceramide microparticles.
FIG. 6B is a graph of a relation between a hole diameter of a small hole and a heat quantity of fusion of ceramide microparticles.
FIG. 7A is a graph of a relation between a content of Surfactant B and an average particle diameter of ceramide microparticles in ceramide microparticle dispersions.
FIG. 7B is a graph of a relation between a content of Surfactant B and a heat quantity of fusion of ceramide microparticles in ceramide microparticle dispersions.

DESCRIPTION OF EMBODIMENTS

An embodiment will be described in detail below.

A method for producing a ceramide microparticle dispersion according to the embodiment includes a component preparation step, a merging step, and a small hole passing step. Specifically, in the method for producing a ceramide microparticle dispersion according to the embodiment, at the component preparation step, a first mixture in a liquid state and a second mixture in a liquid state are prepared, the first mixture containing a ceramide and sterols and having a mass ratio of the content of a water-soluble organic solvent to the content of the ceramide of 0 or more and 30 or less, the second mixture containing water. Thereafter, at the merging step, the first mixture in a liquid state and the liquid second mixture in a liquid state prepared at the component preparation step are caused to flow separately from each other, and then merged together. At the small hole passing step, a fluid formed by merging the first and second mixtures together at the merging step is passed through a small hole having a hole diameter of 0.03 mm or larger and 20 mm or smaller.

The method for producing a ceramide microparticle dispersion according to the embodiment causes the first mixture in a liquid state containing a ceramide and sterols and having a mass ratio of the content of a water-soluble organic solvent to the content of the ceramide of 0 or more and 30 or less and the second mixture in a liquid state containing water to flow separately from each other, merges the first and second mixtures together, and passes the fluid formed by merging the first and second mixtures together through the small hole with a hole diameter of 0.03 mm or more and 20 mm or less, thereby achieving a ceramide microparticle dispersion low in the crystallinity of the ceramide. It is considered that this is because when the fluid formed by merging the first and second mixtures together is passed through the small hole, while the first mixture forms ceramide microparticles, the molecules of sterols are present among ceramide molecules to inhibit crystallization of the ceramide. The term "ceramide microparticles" as used herein means particles of the first mixture, the particles containing the ceramide and dispersed in the second mixture.

The ceramide microparticle dispersion produced by the production method according to this embodiment is low in the crystallinity of the ceramide and can consequently be used for cosmetics, for example. In that case, since the time taken by ceramide crystals to disintegrate is short or unnecessary, the ceramide is expected to instantly penetrate the skin when the cosmetics are applied to the skin.

(Component Preparation Step)

<First Mixture>

The first mixture contains a ceramide and sterols as solid fats. The "solid fats" as used herein refer to oils that are solid at 25°C. In the first mixture in a liquid state, these solid fats have been liquefied by being heated or by addition of a solvent thereto.

The "ceramide" as used herein includes solid ceramides and ceramide analogues with a melting point of 35°C or higher. Specific examples of the ceramide include sugar ceramides, Type I to Type VI natural ceramides, and aliphatic amide derivatives of ceramides such as N-(hexadecyloxyhydroxypropyl)-N-hydroxyethyl hexadecanamide, N-(2-hydroxy-3-hexadecyloxypropyl)-N-2-hydroxyethyl hexadecanamide, N-(2-hydroxy-3-hexadecyloxypropyl)-N-2-hydroxyethyl decanamide, and N-(tetradecyloxyhydroxypropyl)-N-hydroxyethyl decanamide. It is suitable to use one or two or more of them as the ceramide in this embodiment.

The content of the ceramide in the first mixture is, in view of increasing the content of the ceramide in the ceramide microparticle dispersion, suitably 0.1% by mass or more, more suitably 1% by mass or more, even more suitably 5% by mass or more, and still even more suitably 10% by mass or more and is, in view of reducing the crystallinity of the ceramide and reducing the particle diameter of the ceramide microparticles, suitably 30% by mass or less, more suitably 28% by mass or less, even more suitably 25% by mass or less, and still even more suitably 22% by mass or less. The content of the ceramide in the first mixture is suitably 0.1% by mass or more and 30% by mass or less, more suitably 1% by mass or more and 28% by mass or less, even more suitably 5% by mass or more and 25% by mass or less, and still even more suitably 10% by mass or more and 22% by mass or less.

The content of the ceramide in the ceramide microparticle dispersion to be produced is, in view of increasing the content of the ceramide as a moisturizing component, suitably 0.1% by mass or more, more suitably 0.3% by mass or more, and even more suitably 0.5% by mass or more and is, in view of reducing the crystallinity of the ceramide and reducing the particle diameter of the ceramide microparticles, suitably 10% by mass or less, more suitably 5% by mass or less, and even more suitably 3% by mass or less. The content of the ceramide in the ceramide microparticle dispersion to be produced is suitably 0.1% by mass or more and 10% by mass or less, more suitably 0.3% by mass or more and 5% by mass or less, and even more suitably 0.5% by mass or more and 3% by mass or less.

Examples of the sterols include cholesterol, phytosterol, dihydrocholesterol, cholesteryl stearate, cholesteryl nonanoate, cholesteryl hydroxystearate, dihydrocholesteryl oleate, and derivatives and analogues thereof. It is suitable to use one or two or more of them as the sterols in this embodiment.

The content of the sterols in the first mixture is, in view of reducing the crystallinity of the ceramide, suitably 0.5% by mass or more, more suitably 1% by mass or more, and even more suitably 2% by mass or more and is, in view of reducing the particle diameter of the ceramide microparticles, suitably 30% by mass or less, more suitably 20% by mass or less, and even more suitably 15% by mass or less. The content of the sterols in the first mixture is suitably 0.5% by mass or more and 30% by mass or less, more suitably 1% by mass or more and 20% by mass or less, and even more suitably 2% by mass or more and 15% by mass or less.

The content of the sterols in the ceramide microparticle dispersion to be produced is, in view of reducing the crystallinity of the ceramide, suitably 0.1% by mass or more, more suitably 0.2% by mass or more, and even more suitably 0.3% by mass or more and is, in view of reducing the particle diameter of the ceramide microparticles, suitably 5% by mass or less, more suitably 3% by mass or less, and even more suitably 1.5% by mass or less. The content of the sterols in the ceramide microparticle dispersion to be produced is suitably 0.1% by mass or more and 5% by mass or less, more suitably 0.2% by mass or more and 3% by mass or less, and even more suitably 0.3% by mass or more and 1.5% by mass or less.

In each of the first mixture and the ceramide microparticle dispersion to be produced, the content of the sterols is, in view of reducing the crystallinity of the ceramide and reducing the particle diameter of the ceramide microparticles, suitably the same as the content of the ceramide or less than the content of the ceramide. In each of the first mixture and the ceramide microparticle dispersion to be produced, the mass ratio of the content of the sterols to the content of the ceramide (the content of the sterols/the content of the ceramide) is, in view of reducing the crystallinity of the ceramide, suitably 0.10 or more, more suitably 0.20 or more, and even more suitably 0.30 or more and is, in view of reducing the particle diameter of the ceramide microparticles, suitably 5.0 or less, more suitably 3.0 or less, and even more suitably 1.5 or less. In each of the first mixture and the ceramide microparticle dispersion to be produced, the mass ratio of the content of the sterols to the content of the ceramide (the content of the sterols/the content of the ceramide) is suitably 0.1 or more and 5.0 or less, more suitably 0.20 or more and 3.0 or less, and even more suitably 0.30 or more and 1.5 or less.

The first mixture, in view of reducing the crystallinity of the ceramide and obtaining favorable preparation workability, suitably contains a water-soluble organic solvent. The "water-soluble organic solvent" as used herein refers to an organic solvent having a solubility of 1% by mass or more with respect to water at 25°C. Examples of the water-soluble organic solvent include: lower alcohols such as ethanol, propanol, isopropanol, and butanol; polyhydric alcohols such as ethylene glycol, 1,3-propanediol, dipropylene glycol, polybutylene glycol, glycerin, 1,2-propanediol, 1,2-butanediol, 1,4-butanediol, hexanediol, octanediol, hexylene glycol, isoprene glycol, polyethylene glycol, glycerin, diglycerin, triglycerin, polyglycerin, and methyl gluceth; and aliphatic carboxylate esters such as methyl acetate and ethyl acetate. It is suitable to use one or two or more of them as the water-soluble organic solvent in this embodiment.

When the first mixture contains the water-soluble organic solvent, the content thereof is, in view of obtaining favorable preparation workability, suitably more than 0% by mass, more suitably 60% by mass or more, and even more suitably 65% by mass or more and is, in view of reducing the crystallinity of the ceramide, suitably 99% by mass or less, more suitably 95% by mass or less, even more suitably 90% by mass or less, and still even more suitably 75% by mass or less.

When the first mixture contains the water-soluble organic solvent, the mass ratio of the content of the water-soluble organic solvent to the content of the ceramide in the first mixture (the content of the water-soluble organic solvent/the content of the ceramide) is greater than 0. This mass ratio is, in view of obtaining favorable preparation workability, suitably 1.0 or more, more suitably 2.0 or more, even more suitably 3.0 or more, still even more suitably 4.0 or more, and yet still even more suitably 5.0 or more, and is, in view of reducing the crystallinity of the ceramide, suitably 22 or less, more suitably 17 or less, even more suitably 12 or less, and still even more suitably 7.0 or less.

The content of the water-soluble organic solvent in the ceramide microparticle dispersion to be produced is, in view of obtaining favorable preparation workability, suitably 1% by mass or more, more suitably 3% by mass or more, and even more suitably 5% by mass or more and is, in view of reducing the crystallinity of the ceramide, suitably 40% by mass or less, more suitably 20% by mass or less, and even more suitably 15% by mass or less.

The mass ratio of the content of the water-soluble organic solvent to the content of the ceramide in the ceramide microparticle dispersion to be produced (the content of the water-soluble organic solvent/the content of the ceramide) is, in view of obtaining favorable preparation workability, suitably 1.0 or more, more suitably 3.0 or more, and even more suitably 5.0 or more and is, in view of reducing the crystallinity of the ceramide, suitably 30 or less, more suitably 22 or less, even more suitably 17 or less, still even more suitably 12 or less, and yet still even more suitably 7.0 or less.

The first mixture suitably contains a surfactant. Examples of the surfactant include ionic surfactants and nonionic surfactants.

The first mixture, in view of reducing the crystallinity of the ceramide and reducing the particle diameter of the ceramide microparticles, suitably contains a surfactant having a neutralizable functional group. The "surfactant having a neutralizable functional group" as used herein refers to a surfactant that shows almost no ionicity in a non-neutralized state and forms an ionic salt by being mixed with a predetermined neutralizer. Hereinafter, this surfactant having a neutralizable functional group is referred to also as "Surfactant A."

Examples of Surfactant A forming a cationic salt, for example, by being mixed with a predetermined neutralizer include Surfactant A having an amino group and a hydrophobic group.

Examples of the hydrophobic group include a saturated linear hydrocarbon group, a saturated branched hydrocarbon group, an unsaturated linear hydrocarbon group, and an unsaturated branched hydrocarbon group. The number of carbons included in the hydrocarbon group is, in view of reducing the crystallinity of the ceramide and reducing the particle diameter of the ceramide microparticles, suitably 8 or more, more suitably 10 or more, and even more suitably 12 or more and is, in view of the same, suitably 30 or less, more suitably 22 or less, even more suitably 20 or less, and still even more suitably 18 or less. The number of carbons included in the hydrocarbon group is suitably 8 or more and 30 or less, more suitably 10 or more and 22 or less, even more suitably 12 or more and 20 or less, and still even more suitably 12 or more and 18 or less. Specific examples of the hydrocarbon group include: alkyl groups such as a 2-ethylhexyl group, an octyl group, a nonyl group, an isononyl group, a decyl group, an undecyl group, a dodecyl group, a tridecyl group, an isotridecyl group, a tetradecyl group, a pentadecyl group, a hexadecyl group, a heptadecyl group, an octadecyl group, and an isooctadecyl group; and alkenyl groups such as an oleyl group. Among them, in view of reducing the crystallinity of the ceramide and reducing the particle diameter of the ceramide microparticles, a hexadecyl group, a heptadecyl group, an octadecyl group, and an isooctadecyl group are suitable; and an octadecyl group and an isooctadecyl group are more suitable. Surfactant A suitably has one or two or more of these hydrophobic groups.

Examples of Surfactant A having an amino group and a hydrophobic group include primary amine compounds, secondary amine compounds, and tertiary amine compounds.

Examples of the primary amine compounds include long-chain alkylamines. The number of carbons of a long-chain alkyl group of the long-chain alkylamines is, in view of reducing the crystallinity of the ceramide and reducing the particle diameter of the ceramide microparticles, suitably 8 or more, more suitably 10 or more, and even more suitably 12 or more and is, in view of the same, suitably 30 or less, more suitably 22 or less, even more suitably 20 or less, and still even more suitably 18 or less. The number of carbons of the long-chain alkyl group of the long-chain alkylamines is suitably 8 or more and 30 or less, more suitably 10 or more and 22 or less, even more suitably 12 or more and 20 or less, and sill even more suitably 12 or more and 18 or less. The same applies for a suitable number of carbons of the long-chain compounds described below.

Examples of the secondary amine compounds include: dialkyl amines having a long-chain alkyl group and a short-chain alkyl group such as long-chain alkyl methyl amines, long-chain alkyl ethyl amines, and long-chain alkyl propyl amines; and sphingosines (e.g., 4D-hydroxy sphinganine and 1-(2-hydroxyethylamino)-3-isostearyloxy-2-propanol). Examples of the short-chain alkyl group include $C_{1-4}$ alkyl groups such as a methyl group, an ethyl group, a n-propyl group, an i-propyl group, a n-butyl group, an i-butyl group, and a t-butyl group. Specific examples of the short-chain alkyl group described below also include the same.

Examples of the sphingosines include the following (i) to (iv).

[Chemical Formula 1]

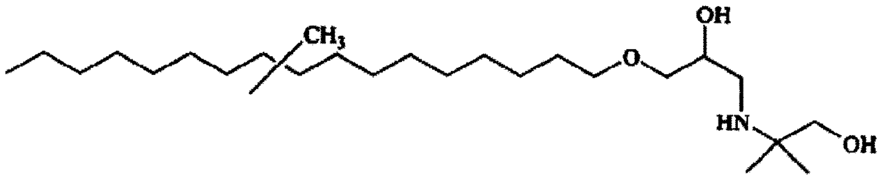
(i)

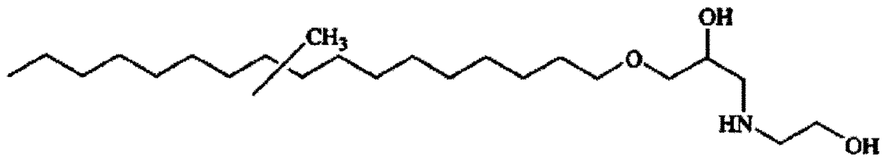
(ii)

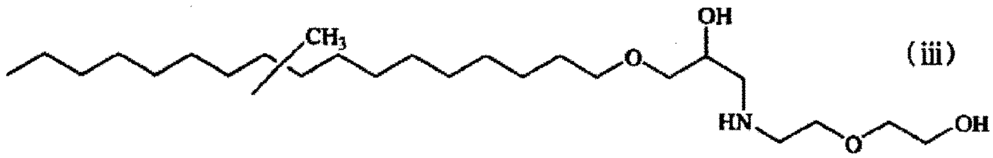
(iii)

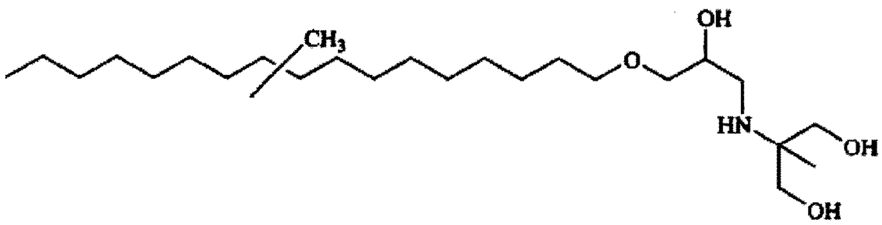
(iv)

Examples of the tertiary amine compounds include: trialkylamines having a long-chain alkyl group and a short-chain alkyl group such as long-chain alkyl dimethyl amines, long-chain alkyl diethyl amines, and long-chain alkyl dipropyl amines; long-chain fatty acid dialkylamino ethylamides and long-chain fatty acid dialkylamino propylamides such as stearic acid dimethylamino propylamide and stearic acid diethylamino ethylamide; arachidyloxy propyl dimethylamines such as hexadecyloxy propyl dimethylamine, stearoxy propyl dimethylamine, stearoxy ethyl dimethylamine, and octadecyloxy propyl dimethylamine (N,N-dimethyl-3-octadecyloxy propylamine); and acyloxy ethyl dialkylamines and acyloxy propyl dialkylamines such as behenyloxy propyl dimethylamine. The number of carbons included in the long-chain fatty acid and the acyl group is, in view of reducing the crystallinity of the ceramide and reducing the particle diameter of the ceramide microparticles, suitably 8 or more, more suitably 10 or more, and even more suitably 12 or more and is, in view of the same, suitably 30 or less, more suitably 22 or less, even more suitably 20 or less, and still even more suitably 18 or less. The number of carbons included in the long-chain fatty acid and the acyl group is suitably 8 or more and 30 or less, more suitably 10 or more and 22 or less, even more suitably 12 or more and 20 or less, and still even more suitably 12 or more and 18 or less. Examples of a hydrocarbon group included in the long-chain fatty acid and the acyl group include groups similar to those exemplified for the hydrophobic group. Examples of an alkyl group included in the dialkyl include $C_{1-4}$ short-chain alkyl groups such as a methyl group, an ethyl group, a n-propyl group, an i-propyl group, a n-butyl group, an i-butyl group, and a t-butyl group.

It is suitable to use one or two or more of them as Surfactant A in this embodiment. In view of reducing the crystallinity of the ceramide and reducing the particle diameter of the ceramide microparticles, it is more suitable to use Surfactant A that forms a cationic salt by being mixed with a predetermined neutralizer. Surfactant A may be partially neutralized to the extent that the effects of reducing the crystallinity of the ceramide and reducing the particle diameter of the ceramide microparticles are not impaired.

The content of Surfactant A in the first mixture is, in view of reducing the crystallinity of the ceramide and reducing the particle diameter of the ceramide microparticles by a low content of Surfactant A, suitably 0.5% by mass or more, more suitably 1% by mass or more, and even more suitably 1.5% by mass or more and is, in view of the same, suitably 20% by mass or less, more suitably 15% by mass or less, and even more suitably 10% by mass or less. The content of Surfactant A in the first mixture is suitably 0.5% by mass or more and 20% by mass or less, more suitably

1% by mass or more and 15% by mass or less, and even more suitably 1.5% by mass or more and 10% by mass or less.

The content of Surfactant A in the ceramide microparticle dispersion to be produced is, in view of reducing the crystallinity of the ceramide and reducing the particle diameter of the ceramide microparticles by a low content of Surfactant A, suitably 0.05% by mass or more, more suitably 0.07% by mass or more, and even more suitably 0.1% by mass or more and is, in view of the same, suitably 2% by mass or less, more suitably 1% by mass or less, and even more suitably 0.5% by mass or less. The content of Surfactant A in the ceramide microparticle dispersion to be produced is suitably 0.05% by mass or more and 2% by mass or less, more suitably 0.07% by mass or more and 1% by mass or less, and even more suitably 0.1% by mass or more and 0.5% by mass or less.

In each of the first mixture and the ceramide microparticle dispersion to be produced, the mass ratio of the content of Surfactant A to the content of the ceramide (the content of Surfactant A/the content of the ceramide) is, in view of reducing the crystallinity of the ceramide and reducing the particle diameter of the ceramide microparticles by a low content of Surfactant A, suitably 0.050 or more, more suitably 0.070 or more, and even more suitably 0.10 or more and is, in view of the same, suitably 2.0 or less, more suitably 1.0 or less, and even more suitably 0.50 or less. In each of the first mixture and the ceramide microparticle dispersion to be produced, the mass ratio of the content of Surfactant A to the content of the ceramide (the content of Surfactant A/the content of the ceramide) is suitably 0.050 or more and 2.0 or less, more suitably 0.070 or more and 1.0 or less, and even more suitably 0.10 or more and 0.50 or less.

In each of the first mixture and the ceramide microparticle dispersion to be produced, the mass ratio of the content of Surfactant A to the content of the sterols (the content of Surfactant A/the content of the sterols) is, in view of reducing the crystallinity of the ceramide and reducing the particle diameter of the ceramide microparticles by a low content of Surfactant A, suitably 0.050 or more, more suitably 0.070 or more, and even more suitably 0.10 or more and is, in view of the same, suitably 3.0 or less, more suitably 2.0 or less, even more suitably 1.0 or less, still even more suitably 0.40 or less, and yet still even more suitably 0.20 or less.

The first mixture, in view of reducing the crystallinity of the ceramide and reducing the particle diameter of the ceramide microparticles, suitably contains a nonionic surfactant (hereinafter, referred to also as "Surfactant B").

Examples of Surfactant B include glycerin fatty acid esters, propylene glycol fatty acid esters, sorbitan fatty acid esters, polyoxyethylene sorbitan fatty acid esters, propylene fatty acid esters, glycerin fatty acid esters, sucrose fatty acid esters, polyoxyethylene sorbitol fatty acid esters, tetraoleic acid polyoxyethylene sorbitol, polyoxyethylene octyl dodecyl ether, polyoxyethylene alkyl ethers, polyoxypropylene alkyl ethers, alkyl glyceryl ethers, polyoxyethylene polyoxypropylene glycol, polyoxyethylene polyoxypropylene alkyl ethers, polyethylene glycol fatty acid esters, polyoxyethylene castor oil, and polyoxyethylene hardened castor oil. It is suitable to use one or two or more of them as Surfactant B in this embodiment.

The hydrophile-lipophile balance (HLB) of Surfactant B is, in view of reducing the crystallinity of the ceramide and reducing the particle diameter of the ceramide microparticles, suitably 2 or more, more suitably 3 or more, and even more suitably 4 or more and is, in view of the same, suitably 15 or less, more suitably 14 or less, and even more suitably 12 or less. The HLB of Surfactant B is suitably 2 or more and 15 or less, more suitably 3 or more and 14 or less, and even more suitably 4 or more and 12 or less.

HLB is determined based on a calculation formula described on pp. 8 to 12 of "Nyuka Kayoka no Gijutsu" Kogaku Tosho (Kabu) (S59-5-20).

More specifically, for a polyhydric alcohol fatty acid ester, HLB is determined based on the following expression:

$$[HLB] = 20(1\text{-}S/A)$$

(wherein S represents the saponification value of the ester, and A represents the acid value of the fatty acid).

For an oxyethylene adduct of a polyhydric alcohol fatty acid ester, HLB is determined based on the following expression:

$$[HLB] = (E + P)/5$$

(wherein E represents an oxyethylene content (% by mass), and P represents a polyhydric alcohol content (% by mass)).

For an oxyethylene adduct of a higher alcohol, HLB is determined based on the following expression:

$$[HLB] = E/5$$

(wherein E is the same as the above).

For Surfactant B other than the above, HLB is determined based on the following expression:

$$[HLB] = 7 + 1.171 \log (Mw/Mo)$$

(wherein Mw represents the molecular weight of a hydrophilic group of the surfactant, Mo represents the molecular weight of a hydrophobic group of the surfactant, and log represents the logarithm to the base 10.).

In the case where two kinds of surfactants, namely, Surfactant X and Surfactant Y, are used in combination as Surfactant B, if the HLB of Surfactant X is denoted by HLBx, the HLB of the Surfactant Y is denoted by $HLB_Y$, and their respective mass fractions are denoted by Wx and $W_Y$, the HLB of Surfactant B formed by mixing them together is determined based on the following expression:

$$[HLB] = [(W_X \times HLB_X) + (W_Y \times HLB_Y)]/(W_X + W_Y)$$

When three or more surfactants are used in combination as Surfactant B, the HLB of Surfactant B formed by mixing them together can be determined in a manner similar to the above.

The content of Surfactant B in the first mixture is, in view of reducing the crystallinity of the ceramide and reducing the particle diameter of the ceramide microparticles by a low content of Surfactant B, suitably 1% by mass or more, more suitably 3% by mass or more, and even more suitably 5% by mass or more and is, in view of the same, suitably 20% by mass or less, more suitably 15% by mass or less, and even more suitably 10% by mass or less. The content of Surfactant B in the first mixture is suitably 1% by mass or more and 20% by mass or less, more suitably 3% by mass or more and 15% by mass or less, and even more suitably 5% by mass or more and 10% by mass or less.

The content of Surfactant B in the ceramide microparticle dispersion to be produced is, in view of reducing the crystallinity of the ceramide and reducing the particle diameter of the ceramide microparticles by a low content of Surfactant B, suitably 0.1% by mass or more, more suitably 0.2% by mass or more, and even more suitably 0.4% by mass or more and is, in view of the same, suitably 2.0% by mass or less, more suitably 1.0% by mass or less, and even more suitably 0.8% by mass or less. The content of Surfactant B in the ceramide microparticle dispersion to be produced is suitably 0.1% by mass or more and 2.0% by mass or less, more suitably 0.2% by mass or more and 1.0% by mass or less, and even more suitably 0.4% by mass or more and 0.8% by mass or less.

In each of the first mixture and the ceramide microparticle dispersion to be produced, the mass ratio of the content of Surfactant B to the content of the ceramide (the content of Surfactant B/the content of the ceramide) is, in view of reducing the crystallinity of the ceramide and reducing the particle diameter of the ceramide microparticles by a low content of Surfactant B, suitably 0.10 or more, more suitably 0.20 or more, and even more suitably 0.40 or more and is, in view of the same, suitably 2.0 or less, more suitably 1.0 or less, and even more suitably 0.80 or less. In each of the first mixture and the ceramide microparticle dispersion to be produced, the mass ratio of the content of Surfactant B to the content of the ceramide (the content of Surfactant B/the content of the ceramide) is suitably 0.10 or more and 2.0 or less, more suitably 0.20 or more and 1.0 or less, and even more suitably 0.40 or more and 0.80 or less.

In each of the first mixture and the ceramide microparticle dispersion to be produced, the mass ratio of the content of Surfactant B to the content of the sterols (the content of Surfactant B/the content of the sterols) is, in view of reducing the crystallinity of the ceramide and reducing the particle diameter of the ceramide microparticles by a low content of Surfactant B, suitably 0.10 or more, more suitably 0.50 or more, and even more suitably 1.0 or more and is, in view of the same, suitably 10 or less, more suitably 5.0 or less, and even more suitably 3.0 or less.

The first mixture, in view of reducing the crystallinity of the ceramide and reducing the particle diameter of the ceramide microparticles, suitably contains both Surfactant A and Surfactant B.

When the first mixture contains both Surfactant A and Surfactant B, the sum of the contents of Surfactant A and Surfactant B in the first mixture is, in view of reducing the crystallinity of the ceramide and reducing the particle diameter of the ceramide microparticles by low contents of Surfactant A and Surfactant B, suitably 3% by mass or more, more suitably 5% by mass or more, and even more suitably 7% by mass or more and is, in view of the same, suitably 25% by mass or less, more suitably 18% by mass or less, and even more suitably 12% by mass or less. The sum of the contents of Surfactant A and Surfactant B in the first mixture is suitably 3% by mass or more and 25% by mass or less, more suitably 5% by mass or more and 18% by mass or less, and even more suitably 7% by mass or more and 12% by mass or less.

When the first mixture contains both Surfactant A and Surfactant B, the sum of the contents of Surfactant A and Surfactant B in the ceramide microparticle dispersion to be produced is, in view of reducing the crystallinity of the ceramide and reducing the particle diameter of the ceramide microparticles by low contents of Surfactant A and Surfactant B, suitably 0.1% by mass or more, more suitably 0.2% by mass or more, and even more suitably 0.4% by mass or more

and is, in view of the same, suitably 3.0% by mass or less, more suitably 2.0% by mass or less, and even more suitably 1.0% by mass or less. The sum of the contents of Surfactant A and Surfactant B in the ceramide microparticle dispersion to be produced is suitably 0.1% by mass or more and 3.0% by mass or less, more suitably 0.2% by mass or more and 2.0% by mass or less, and even more suitably 0.4% by mass or more and 1.0% by mass or less.

When the first mixture contains both Surfactant A and Surfactant B, in each of the first mixture and the ceramide microparticle dispersion to be produced, the content of Surfactant B is, in view of reducing the crystallinity of the ceramide and reducing the particle diameter of the ceramide microparticles, suitably greater than the content of Surfactant A. In each of the first mixture and the ceramide microparticle dispersion to be produced, the mass ratio of the content of Surfactant B to the content of Surfactant A (Surfactant B/Surfactant A) is, in view of reducing the crystallinity of the ceramide and reducing the particle diameter of the ceramide microparticles, suitably 0.50 or more, more suitably 1.0 or more, and even more suitably 2.0 or more and is, in view of the same, suitably 10 or less, more suitably 8.0 or less, and even more suitably 5.0 or less. In each of the first mixture and the ceramide microparticle dispersion to be produced, the mass ratio of the content of Surfactant B to the content of Surfactant A (Surfactant B/Surfactant A) is suitably 0.50 or more and 10 or less, more suitably 1.0 or more and 8.0 or less, and even more suitably 2.0 or more and 5.0 or less.

When the first mixture contains both Surfactant A and Surfactant B, in each of the first mixture and the ceramide microparticle dispersion to be produced, the mass ratio of the sum of the contents of Surfactant A and Surfactant B to the content of the ceramide (the sum of the contents of Surfactant A and Surfactant B/the content of the ceramide) is, in view of reducing the crystallinity of the ceramide and reducing the particle diameter of the ceramide microparticles by low contents of Surfactant A and Surfactant B, suitably 0.15 or more, more suitably 0.27 or more, and even more suitably 0.50 or more and is, in view of the same, suitably 4.0 or less, more suitably 2.0 or less, and even more suitably 1.0 or less.

When the first mixture contains both Surfactant A and Surfactant B, in each of the first mixture and the ceramide microparticle dispersion to be produced, the mass ratio of the sum of the contents of Surfactant A and Surfactant B to the content of the sterols (the sum of the contents of Surfactant A and Surfactant B/the content of the sterols) is, in view of reducing the crystallinity of the ceramide and reducing the particle diameter of the ceramide microparticles by low contents of Surfactant A and Surfactant B, suitably 0.50 or more, more suitably 1.0 or more, and even more suitably 2.0 or more and is, in view of the same, suitably 10 or less, more suitably 6.0 or less, and even more suitably 4.0 or less.

The first mixture may additionally contain a solid fat other than the ceramide and the sterols, and a liquid oil such as an oil that is in a liquid state at 25°C.

<Second Mixture>

The second mixture is a mixture including water as a main component. The second mixture suitably incudes ion-exchange water or distilled water. The second mixture may contain a water-soluble organic solvent in addition to water. Examples of the water-soluble organic solvent include ethanol, ethylene glycol, and glycerin. The content of the solvent in the dispersion to be produced is 1% by mass or more and 20% by mass or less, for example.

When the first mixture contains Surfactant A having a neutralizable functional group, the second mixture suitably contains a neutralizer that is capable of forming a salt with Surfactant A. The "neutralizer that is capable of forming a salt with Surfactant A" as used herein refers to an acidic compound or a basic compound that is capable of forming a salt with Surfactant A.

When Surfactant A is Surfactant A having an amino group and a hydrophobic group, the neutralizer is suitably an acidic compound.

Examples of the acidic compound as the neutralizer include inorganic acids and organic acids. Examples of the inorganic acids include phosphoric acid, hydrochloric acid, nitric acid, sulfuric acid, perchloric acid, and carbonic acid. Examples of the organic acids include: monocarboxylic acids such as formic acid, acetic acid, propionic acid, butyric acid, iso-butyric acid, and valeric acid; dicarboxylic acids such as succinic acid, phthalic acid, fumaric acid, oxalic acid, malonic acid, and glutaric acid; oxycarboxylic acids such as glycolic acid, citric acid, lactic acid, pyruvic acid, malic acid, and tartaric acid; and amino acids such as L-glutamic acid and aspartic acid. The number of carbons included in the organic acid is, in view of reducing the crystallinity of the ceramide and reducing the particle diameter of the ceramide microparticles, suitably 2 or more and more suitably 3 or more, and is suitably 7 or less and more suitably 6 or less. The number of carbons included in the organic acid is suitably 2 or more and 7 or less and more suitably 3 or more and 6 or less. It is suitable to use one or two or more of them as the acidic compound serving as the neutralizer in this embodiment. In view of reducing the crystallinity of the ceramide and reducing the particle diameter of the ceramide microparticles, the organic acid is suitably used as the acidic compound serving as the neutralizer. At least one of the amino acid or the oxycarboxylic acid is more suitably used, and at least one of L-glutamic acid or lactic acid is even more suitably used.

The content of the neutralizer in the second mixture is, in view of reducing the crystallinity of the ceramide and reducing the particle diameter of the ceramide microparticles, suitably 0.05% by mass or more, more suitably 0.08% by mass or more, and even more suitably 0.1% by mass or more and is, in view of the same, suitably 1.0% by mass or

less, more suitably 0.5% by mass or less, and even more suitably 0.2% by mass or less. The content of the neutralizer in the second mixture is suitably 0.05% by mass or more and 1.0% by mass or less, more suitably 0.08% by mass or more and 0.5% by mass or less, and even more suitably 0.1% by mass or more and 0.2% by mass or less.

The content of the neutralizer in the ceramide microparticle dispersion to be produced is, in view of reducing the crystallinity of the ceramide and reducing the particle diameter of the ceramide microparticles, suitably 0.03% by mass or more, more suitably 0.05% by mass or more, and even more suitably 0.08% by mass or more and is, in view of the same, suitably 1.0% by mass or less, more suitably 0.5% by mass or less, and even more suitably 0.2% by mass or less. The content of the neutralizer in the ceramide microparticle dispersion to be produced is suitably 0.03% by mass or more and 1.0% by mass or less, more suitably 0.05% by mass or more and 0.5% by mass or less, and even more suitably 0.08% by mass or more and 0.2% by mass or less.

The second mixture may contain a surfactant. However, in view of reducing the crystallinity of the ceramide and reducing the particle diameter of the ceramide microparticles, it is suitable that the second mixture contains substantially no surfactant. The content of the surfactant contained in the second mixture is, in terms of a content in the ceramide microparticle dispersion to be produced, suitably 1.0% by mass or less, more suitably 0.5% by mass or less, and even more suitably 0.1% by mass or less. The second mixture may additionally contain an antiseptic or the like.

(Merging Step)

At the merging step, the first mixture in a liquid state and the second mixture in a liquid state are merged, the first and second mixtures having been caused to flow separately from each other.

A ratio of a flow rate $Q_1$ of the first mixture to a flow rate $Q_2$ of the second mixture before being merged ($Q_1/Q_2$) is, in view of increasing the content of the ceramide, suitably 1/99 or more, more suitably 3/97 or more, and even more suitably 5/95 or more and is, in view of reducing the crystallinity of the ceramide and reducing the particle diameter of the ceramide microparticles, suitably 30/70 or less, more suitably 25/75 or less, and even more suitably 20/80 or less. The ratio of the flow rate $Q_1$ of the first mixture to the flow rate $Q_2$ of the second mixture before being merged ($Q_1/Q_2$) is suitably 1/99 or more and 30/70 or less, more suitably 3/97 or more and 25/75 or less, and even more suitably 5/95 or more and 20/80 or less.

The temperature of the first mixture before being merged is, in view of reducing the crystallinity of the ceramide and reducing the particle diameter of the ceramide microparticles, suitably 60°C or higher, more suitably 70°C or higher, and even more suitably 80°C or higher and is, in view of the same, suitably 120°C or lower, more suitably 110°C or lower, and even more suitably 100°C or lower. The temperature of the first mixture before being merged is suitably 60°C or higher and 120°C or lower, more suitably 70°C or higher and 110°C or lower, and even more suitably 80°C or higher and 100°C or lower.

The temperature of the second mixture before being merged is, in view of reducing the crystallinity of the ceramide and reducing the particle diameter of the ceramide microparticles, suitably 10°C or higher, more suitably 20°C or higher, and even more suitably 30°C or higher and is, in view of the same, suitably 90°C or lower, more suitably 80°C or lower, and even more suitably 70°C or lower. The temperature of the second mixture before being merged is suitably 10°C or higher and 90°C or lower, more suitably 20°C or higher and 80°C or lower, and even more suitably 30°C or higher and 70°C or lower.

Before being merged, the first mixture may have the same temperature as that of the second mixture. However, in view of reducing the crystallinity of the ceramide and reducing the particle diameter of the ceramide microparticles, the first mixture suitably has a higher temperature than the second mixture.

At the merging step, the first and second mixtures may be merged in any manner.

Examples of the manner (angle and the like) of collision when the first mixture and the second mixture are merged together include a manner in which the first and second mixtures are caused to collide head-on with each other so as to be merged together (counterflow collision), a manner in which one of the first and second mixtures is caused to collide with the other in a direction perpendicular to the other so as to be merged therewith, a manner in which one of the first and second mixtures is caused to collide with the other obliquely in the flow direction of the one so that the first and second mixtures are merged together, a manner in which one of the first and second mixtures is caused to collide with the other obliquely in a direction opposite to the flow direction of the one so that the first and second mixtures are merged together, a manner in which one of the first and second mixtures is caused to flow along, and come into contact with, the other so as to be merged therewith, and a manner in which one of the first and second mixtures is caused to collide with the other while the one comes into contact with the entire periphery of the other so as to be merged therewith. Among them, the manner in which one of first and second mixtures is caused to collide with the other while the one comes into contact with the entire periphery of the other so that the first and second mixtures are merged together is suitable.

Examples of the manner (number and the like) of the way of merging the first mixture and the second mixture together include a manner in which the first and the second mixtures are caused to collide with each other in a plurality

of directions each so as to be merged together and a manner in which one of the first and the second mixtures is caused to collide with the other in a plurality of directions to be merged therewith. In the former, both of them are suitably caused to collide with each other in two or more directions each; although the upper limit is not limited to a particular number, both of them are suitably caused to collide with each other in four or less directions each in view of productivity. In the latter, one of them is suitably caused to collide with the other in two or more directions; although the upper limit is not limited to a particular number, one of them is suitably caused to collide with the other in four or less directions in view of productivity.

The first and second mixtures may be merged together after or concurrently with passing of at least one of them through a small hole having a configuration similar to that of the small hole through which a fluid formed by merging the first and second mixtures together will be passed at the small hole passing step to be described below.

(Small Hole Passing Step)

At the small hole passing step, the fluid formed by merging the first and second mixtures together at the merging step is passed through a small hole having a hole diameter of 0.03 mm or larger and 20 mm or smaller.

Examples of the cross-sectional shape of the small hole include a circle, a semicircle, an ellipse, a semiellipse, a square, a rectangle, a trapezoid, a parallelogram, a star shape, and an indefinite shape. Among these, a circle is suitable. The cross-sectional shape of the small hole is suitably the same along its depth.

The direction in which the small hole extends is suitably the same as at least one of the flow direction of the first mixture or the flow direction of the second mixture.

The hole diameter of the small hole is 0.03 mm or larger and 20 mm or smaller. In the case of small-volume production equipment, the hole diameter of the small hole is, in view of reducing an operating pressure and increasing a liquid amount to be processed, suitably 0.04 mm or larger, more suitably 0.05 mm or larger, even more suitably 0.1 mm or larger, and still even more suitably 0.15 mm or larger and is, in view of reducing the crystallinity of the ceramide and reducing the particle diameter of the ceramide microparticles, suitably 10 mm or smaller, more suitably 7 mm or smaller, even more suitably 3 mm or smaller, and still even more suitably 1 mm or smaller. The hole diameter of the small hole is suitably 0.04 mm or larger and 10 mm or smaller, more suitably 0.05 mm or larger and 7 mm or smaller, even more suitably 0.1 mm or larger and 3 mm or smaller, and still even more suitably 0.15 mm or larger and 1 mm or smaller. In the case of large-volume production equipment, the hole diameter of the small hole is, in view of reducing an operating pressure and increasing a liquid amount to be processed, suitably 2.0 mm or larger, more suitably 4.0 mm or larger, and even more suitably 6.0 mm or larger and is, in view of reducing the crystallinity of the ceramide and reducing the particle diameter of the ceramide microparticles, suitably 20 mm or smaller, more suitably 18 mm or smaller, and even more suitably 16 mm or smaller. The hole diameter of the small hole is suitably 2.0 mm or larger and 20 mm or smaller, more suitably 4.0 mm or larger and 18 mm or smaller, and even more suitably 6.0 mm or larger and 16 mm or smaller. The hole diameter of the small hole is a diameter when the cross-sectional shape of the small hole is a circle, whereas the hole diameter of the small hole is an equivalent hydraulic diameter (4 $\times$ the flow channel area/the sectional length) when the cross-sectional shape of the small hole is a non-circle. The hole diameter of the small hole is a diameter when the cross-sectional shape of the small hole is a circle whereas the hole diameter of the small hole is an equivalent hydraulic diameter (4 $\times$ the flow channel area/the sectional length) when the cross-sectional shape of the small hole is a non-circle.

The depth of the small hole is, in view of reducing the crystallinity of the ceramide and reducing the particle diameter of the ceramide microparticles, suitably 0.05 mm or greater, more suitably 0.1 mm or greater, even more suitably 0.3 mm or greater, still even more suitably 0.4 mm or greater, and yet still even more suitably 0.5 mm or greater and is, in view of reducing an operating pressure and increasing a liquid amount to be processed, suitably 30 mm or less, more suitably 17 mm or less, even more suitably 9 mm or less, still even more suitably 3 mm or less, and yet still even more suitably 1 mm or less. The depth of the small hole is suitably 0.05 mm or greater and 30 mm or less, more suitably 0.1 mm or greater and 17 mm or less, even more suitably 0.3 mm or greater and 9 mm or less, still even more suitably 0.4 mm or greater and 3 mm or less, and yet still even more suitably 0.5 mm or greater and 1 mm or less.

The ratio of the depth to the hole diameter of the small hole (the depth/the hole diameter) is, in view of reducing the crystallinity of the ceramide and reducing the particle diameter of the ceramide microparticles, suitably 0.10 or more, more suitably 0.50 or more, and even more suitably 1.0 or more and is, in view of reducing an operating pressure and increasing a liquid amount to be processed, suitably 30 or less, more suitably 10 or less, and even more suitably 5.0 or less. The ratio of the depth to the hole diameter of the small hole (the depth/the hole diameter) is suitably 0.10 or more and 30 or less, more suitably 0.50 or more and 10 or less, and even more suitably 1.0 or more and 5.0 or less.

The flow channel area of the small hole is, in view of reducing an operating pressure and increasing a liquid amount to be processed, suitably 0.005 $mm^2$ or larger, more suitably 0.01 $mm^2$ or larger, even more suitably 0.02 $mm^2$ or larger, and still even more suitably 0.05 $mm^2$ or larger and is, in view of reducing the crystallinity of the ceramide and reducing the particle diameter of the ceramide microparticles, suitably 180 $mm^2$ or smaller, more suitably 70 $mm^2$ or

smaller, even more suitably 8 $mm^2$ or smaller, and still even more suitably 1 $mm^2$ or smaller. The flow channel area of the small hole is suitably 0.005 $mm^2$ or larger and 180 $mm^2$ or smaller, more suitably 0.01 $mm^2$ or larger and 70 $mm^2$ or smaller, even more suitably 0.02 $mm^2$ or larger and 8 $mm^2$ or smaller, and still even more suitably 0.05 $mm^2$ or larger and 1 $mm^2$ or smaller.

The method for passing the fluid formed by merging the first and second mixtures together through the small hole is, in view of reducing the crystallinity of the ceramide and reducing the particle diameter of the ceramide microparticles, suitably a method that merges the first and second mixtures together immediately before the small hole and passes the fluid immediately after the merging as it is through the small hole or a method that merges the first and second mixtures together within the small hole and passes the fluid after the merging continuously through the small hole. Alternatively, the first and second mixtures may once be merged together in a liquid reservoir, and a fluid in which the first and second mixtures are in a mixed state may be passed through the small hole.

An average flow velocity u of the fluid passing through the small hole is, in view of reducing the crystallinity of the ceramide and reducing the particle diameter of the ceramide microparticles, suitably 5 m/s or greater, more suitably 10 m/s or greater, and even more suitably 15 m/s or greater and is, in view of the same, suitably 70 m/s or less, more suitably 50 m/s or less, and even more suitably 30 m/s or less. The average flow velocity u of the fluid passing through the small hole is suitably 5 m/s or greater and 70 m/s or less, more suitably 10 m/s or greater and 50 m/s or less, and even more suitably 15 m/s or greater and 30 m/s or less. The average flow velocity u of the fluid passing through the small hole can be controlled with a flow rate Q of the fluid passing through the small hole.

In view of reducing the crystallinity of the ceramide and reducing the particle diameter of the ceramide microparticles, the fluid suitably passes through the small hole in turbulence conditions. The Reynolds number Re of the fluid passing through the small hole is, in view of reducing the crystallinity of the ceramide and reducing the particle diameter of the ceramide microparticles, suitably 3,000 or greater, more suitably 5,000 or greater, and even more suitably 8,000 or greater and is, in view of reducing an operating pressure and increasing a liquid amount to be processed, suitably 300,000 or less, more suitably 250,000 or less, and even more suitably 200,000 or less. The Reynolds number Re of the fluid passing through the small hole is suitably 3,000 or greater and 300,000 or less, more suitably 5,000 or greater and 250,000 or less, and even more suitably 8,000 or greater and 200,000 or less. The Reynolds number Re of the fluid passing through the small hole is determined by the Reynolds number formula for a flow in a general pipe, using the values of the average flow velocity u (m/s) of the fluid within the small hole, the hole diameter d (m) of the small hole, a viscosity $\mu$ (Pa·s) of the fluid, and a density $\rho$ (kg/$m^3$) of the fluid (Reynolds number $Re = du\rho/\mu$).

A pressure loss $\Delta P$ between a pressure upstream of the small hole and a pressure downstream of the small hole refers to a pressure difference between the fluid before passing through the small hole and the fluid after passing through the small hole, by which the magnitude of a jet can be evaluated. The pressure loss $\Delta P$ is, in view of reducing the crystallinity of the ceramide and reducing the particle diameter of the ceramide microparticles, suitably 0.050 MPa or greater, more suitably 0.1 MPa or greater, and even more suitably 0.2 MPa or greater and is, in view of preventing a failure occurring in equipment due to a produced excessive pressure loss $\Delta P$, suitably 5.0 MPa or less, more suitably 2.0 MPa or less, and even more suitably 1.0 MPa or less. The pressure loss $\Delta P$ is suitably 0.050 MPa or greater and 5.0 MPa or less, more suitably 0.1 MPa or greater and 2.0 MPa or less, and even more suitably 0.2 MPa or greater and 1.0 MPa or less.

In view of reducing the crystallinity of the ceramide and reducing the particle diameter of the ceramide microparticles, the fluid that has passed through the small hole is suitably caused to flow out to a flow channel expanded part having an increased flow channel diameter to facilitate the occurrence of turbulence.

Examples of the cross-sectional shape of the flow channel of the flow channel expanded part include a circle, a semicircle, an ellipse, a semiellipse, a square, a rectangle, a trapezoid, a parallelogram, a star shape, and an indefinite shape. Among these, a circle is suitable. The flow channel expanded part suitably has the same cross-sectional shape along its length but may include a portion in a different cross-sectional shape.

In view of reducing the crystallinity of the ceramide and reducing the particle diameter of the ceramide microparticles, the flow channel expanded part is suitably formed to expand in a cone shape from the small hole to a portion having a maximum flow channel diameter. This cone expansion angle ($\theta_{12}$ in FIG. 2A) is, in view of reducing the crystallinity of the ceramide and reducing the particle diameter of the ceramide microparticles, suitably 80° or greater, more suitably 90° or greater, and even more suitably 100° or greater and is, in view of the same, suitably 180° or less, more suitably 170° or less, and even more suitably 140° or less. This cone expansion angle is suitably 80° or greater and 180° or less, more suitably 90° or greater and 170° or less, and even more suitably 100° or greater and 140° or less.

The maximum flow channel diameter of the flow channel expanded part is, in view of reducing the crystallinity of the ceramide and reducing the particle diameter of the ceramide microparticles, suitably two times or more, more suitably three times or more, and even more suitably four times or more the hole diameter of the small hole and is, in view of the same, suitably 20 times or less, more suitably 12 times or less, and even more suitably 10 times or less the hole diameter of the small hole. The maximum flow channel diameter of the flow channel expanded part is suitably two times or more and 20 times or less, more suitably three times or more and 12 times or less, and even more suitably four

times or more and 10 times or less the hole diameter of the small hole. The maximum flow channel diameter of the flow channel expanded part is a diameter when the cross-sectional shape of the flow channel is a circle but is an equivalent hydraulic diameter when the cross-sectional shape of the flow channel is a non-circle.

The operation to pass the fluid formed by merging the first and second mixtures together through the small hole is, in view of reducing the crystallinity of the ceramide and reducing the particle diameter of the ceramide microparticles, suitably performed under a condition such that a segregation index (Xs) determined by a method described below is suitably 0.1 or less, more suitably 0.01 or less, and even more suitably 0.004 or less. The lower limit thereof, which is not limited to a particular value, is suitably at least 0.0001 and may be 0.001 or more.

The segregation index (Xs) indicates the effect of mixing and stirring the first mixture and the second mixture in the method for producing the ceramide microparticle dispersion according to the embodiment; a smaller value indicates a higher degree of mixing performance. The segregation index (Xs) is an indicator used academically and universally for the evaluation of the mixing performance of mixers, and can be derived from the following expression using the Villermaux/Dushman reaction. The details of this reaction system are described in P. Guichardon and L. Falk, Chemical Engineering Science 55 (2000) 4233-4243.

Specifically, first, 0.171 N sulfuric acid and a boric acid buffer solution are prepared as two aqueous solutions to be reacted with each other by mixing. The boric acid buffer solution is prepared so as to have a composition of 0.045 mol/L boric acid, 0.045 mol/L sodium hydroxide, 0.00313 mol/L potassium iodate, and 0.0156 mol/L potassium iodide. When these two liquids are mixed together, the following neutralization reaction (I) and oxidation-reduction reaction (II) to produce iodine proceed simultaneously. The mixing of the two liquids is performed under a condition such that the boric acid buffer solution is excessive after the mixing.

[Chemical Formula 2]

(I) $H+H_2BO^-_3 \leftrightarrow H_3BO_3$

(II) $6H^+ + 5I^- + IO^-_3 \leftrightarrow 3I_2 + 3H_2O$

(III) $I_2 + I^- \leftrightarrow I^-_3$

$$H^+ + H_2BO_3^- \leftrightarrow H_3BO_3 \quad \cdots(\mathrm{I})$$

$$6H^+ + 5I^- + IO_3^- \leftrightarrow 3I_2 + 3H_2O \quad \cdots(\mathrm{II})$$

$$I_2 + I^- \leftrightarrow I_3^- \quad \cdots(\mathrm{III})$$

$$X_S = \frac{Y}{Y_{ST}} \quad Y = \frac{2(n_{I_2} + n_{I_3^-})}{n_{H_0^+}} = 2 \cdot \frac{V_{\text{after mixing}}}{V_{\text{aqueous sulfuric acid solution}}} \cdot \frac{[I_2] + [I_3^-]}{[H^+]_0} \quad \cdots(\mathrm{IV})$$

$$Y_{ST} = \frac{6[IO_3^-]_0}{6[IO_3^-]_0 + [H_2BO_3^-]_0}$$

In this process, since an amount of produced iodine decreases as the time required by the completion of the mixing of the two liquids is shortened, $I_3^-$ ions in a chemical equilibrium relation (III) with the produced iodine are quantified at an absorption wavelength of 353 nm. The segregation index (Xs) given by Expression (IV) is calculated from the quantification result, and mixing performance can be evaluated based the segregation index (Xs). In this process, Expression (V) is used for quantification of $I_3$ ions, and Expressions (VI), (VII), and (VIII) are used for calculation of the concentration of $I_2$.

[Chemical Formula 3]

[Chemical Formula 3]

$$[I_3^-] = \frac{ABS}{\varepsilon \times L} \quad \cdots (V)$$

$$K_B = \frac{[I_3^-]}{[I_2][I^-]} \quad \cdots (VI)$$

$$\log_{10} K_B = \frac{555}{T} + 7.355 - 2.575 \log_{10} T \quad \cdots (VII)$$

$$[I^-] = [I^-]_0 - \frac{5}{3}([I_2] + [I_3^-]) - [I_3^-] \quad \cdots (VIII)$$

The segregation index (Xs) is the proportion of acid consumed by the oxidation-reduction reaction (II) with respect to charged acid. Therefore, it can be evaluated that a smaller value of the segregation index (Xs) indicates higher mixing performance.

ABS represents an absorbance, ε represents a molar extinction coefficient ($m^2$/mol), L represents an optical path length (m), $K_B$ represents an equilibrium constant (L/mol) of the reaction represented by Formula (III), and T represents an absolute temperature (K). $[I_3^-]$, $[I_2]$, and $[I^-]$ represent concentrations of the respective chemical species after mixing, and $[H^+]_0$, $[IO_3^-]_0$, $[H_2BO_3^-]_0$, and $[I^-]_0$ represent the initial concentrations of the respective chemical species. $n_{I2}$ and $n_{I3-}$ represent the numbers of moles of the respective chemical species after mixing, and $n_{H0+}$ represents the initial number of moles of $H^+$. $V_{aqueous\ sulfuric\ acid\ solution}$ represents a volume flow rate of 0.171 N sulfuric acid, and $V_{after\ mixing}$ represents a volume flow rate after mixing the two liquids (the boric acid buffer solution and 0.171 N sulfuric acid) together. When the value of absorbance is out of the measurement range of an absorbance meter due to an excessively high concentration of $I_3^-$ ions, the solution after mixing is diluted with ion-exchange water as appropriate, and then the absorbance is measured.

The fluid that has passed through the small hole is cooled so that the ceramide microparticles are solidified. Thus, the fluid turns into a ceramide microparticle dispersion.

The average particle diameter of the ceramide microparticles in the obtained ceramide microparticle dispersion is suitably 100 nm or smaller, more suitably 80 nm or smaller, and even more suitably 60 nm or smaller. The "average particle diameter" as used herein is a cumulant diameter measured by photon correlation method (dynamic light scattering) using a particle size distribution measurement apparatus.

The heat quantity of fusion of the ceramide microparticles in the obtained ceramide microparticle dispersion in a temperature range of 45°C or higher and 80°C or lower is suitably 0.30 J/g or less, more suitably 0.2 J/g or less, and even more suitably 0.1 J/g or less. This heat quantity of fusion is determined from a total endotherm in a temperature range of 45°C or higher and 80°C or lower of a melting profile obtained by measurement using a differential scanning calorimetric analyzer.

The thus produced ceramide microparticle dispersion is used as a component of cosmetics, for example.

[Micromixer System S]

FIG. 1 illustrates an example of a micromixer system S that is usable in the method for producing a dispersion according to the embodiment. In the following, one of first and second liquids is the first mixture, whereas the other is the second mixture.

The micromixer system S includes a micromixer 100 and auxiliary parts such as a fluid supply system.

FIGS. 2A and 2B illustrate the micromixer 100 of a first configuration.

The micromixer 100 of the first configuration includes a fluid flow channel part 10, a fluid merging-contracting part 20 that is continuous with, and provided downstream of the fluid flow channel part 10, and a fluid outflow part 30 that is continuous with, and provided downstream of the fluid merging-contracting part 20.

The fluid flow channel part 10 has a small-diameter pipe 11 and a large-diameter pipe 12. The small-diameter pipe 11 extends inside the large-diameter pipe 12. These pipes 11 and 12 share a common length direction and are arranged coaxially with each other. With this configuration, the fluid flow channel part 10 includes a first flow channel 11a provided within the small-diameter pipe 11, and a second flow channel 12a provided inside the large-diameter pipe 12 and outside the small-diameter pipe 11. The first flow channel 11a within the small-diameter pipe 11 communicates

with a first liquid supply part 101 provided at one end of the apparatus, whereas the second flow channel 12a within the large-diameter pipe 12 communicates with a second liquid supply part 102 provided on a side face of the apparatus.

The cross-sectional shapes of the outside shape and the hole of the small-diameter pipe 11 are not limited to any particular shape; they may be a circle, a semicircle, an ellipse, a semiellipse, a square, a rectangle, a trapezoid, a parallelogram, a star shape, or an indefinite shape, for example. The cross-sectional shape of the hole of the large-diameter pipe 12 is not limited to any particular shape neither; similarly to the small-diameter pipe 11, it may be a circle, a semicircle, an ellipse, a semiellipse, a square, a rectangle, a trapezoid, a parallelogram, a star shape, or an indefinite shape, for example. However, the cross-sectional shapes of all of the outer shape and the hole of the small-diameter pipe 11 and the hole of the large-diameter pipe 12 are suitably a circle. In addition, the small-diameter pipe 11 and the large-diameter pipe 12 are suitably provided such that their cross-sectional shapes are symmetric and coaxial. Consequently, as illustrated in FIG. 2B, the small-diameter pipe 11 and the large-diameter pipe 12 are suitably provided such that the first flow channel 11a has a circular shape in cross section and the second flow channel 12a has a donut shape in cross section.

The cross-sectional shapes of both of the outer shape and the hole of the small-diameter pipe 11 are suitably the same along its length except a pipe end part 11b described below. The cross-sectional shape of the hole of the large-diameter pipe 12 is also suitably the same along its length except a part corresponding to the pipe end part 11b of the small-diameter pipe 11.

When the cross-sectional shapes of both of the outer shape and the hole of the small-diameter pipe 11 are a circle, its outer diameter $D_{11}$ is suitably 1.6 mm or larger and more suitably 2 mm or larger and is suitably 25 mm or smaller, more suitably 15 mm or smaller, even more suitably 10 mm or smaller, and till even more suitably 4 mm or smaller. The outer diameter $D_{11}$ is suitably 1.6 mm or larger and 25 mm or smaller, more suitably 2 mm or larger and 15 mm or smaller, even more suitably 2 mm or larger and 10 mm or smaller, and still even more suitably 2 mm or larger and 4 mm or smaller.

The inner diameter $D_{12}$ of the small-diameter pipe 11, that is, the flow channel diameter of the first flow channel 11a is suitably 0.8 mm or larger and more suitably 1 mm or larger and is suitably 20 mm or smaller, more suitably 10 mm or smaller, even more suitably 5 mm or smaller, and still even more suitably 3 mm or smaller. The inner diameter $D_{12}$ is suitably 0.8 mm or larger and 20 mm or smaller, more suitably 1 mm or larger and 10 mm or smaller, even more suitably 1 mm or larger and 5 mm or smaller, and still even more suitably 1 mm or larger and 3 mm or smaller.

When the cross-sectional shape of the hole of the large-diameter pipe 12 is a circle, its inner diameter $D_{13}$ is suitably 1.8 mm or larger and more suitably 4 mm or larger and is suitably 50 mm or smaller, more suitably 20 mm or smaller, even more suitably 10 mm or smaller, and still even more suitably 6 mm or smaller. The inner diameter $D_{13}$ is suitably 1.8 mm or larger and 50 mm or smaller, more suitably 4 mm or larger and 20 mm or smaller, even more suitably 4 mm or larger and 10 mm or smaller, and still even more suitably 4 mm or larger and 6 mm or smaller.

The second flow channel 12a between the small-diameter pipe 11 and the large-diameter pipe 12 has a clearance $\Delta_1$ that is suitably 0.1 mm or larger, more suitably 0.3 mm or larger, and even more suitably 0.7 mm or larger and is suitably 12.5 mm or smaller, more suitably 6 mm or smaller, even more suitably 3 mm or smaller, and still even more suitably 1 mm or smaller. The clearance $\Delta_1$ is suitably 0.1 mm or larger and 12.5 mm or smaller, more suitably 0.3 mm or larger and 6 mm or smaller, even more suitably 0.7 mm or larger and 3 mm or smaller, and still even more suitably 0.7 mm or larger and 1 mm or smaller.

As illustrated in FIG. 2A, the pipe end part 11b located in a downstream portion of the small-diameter pipe 11 is suitably formed such that its outer periphery is tapered and is more suitably formed such that its cross-sectional shape in the thickness direction has the shape of a spire whose narrow end is close to the inner periphery of the small-diameter pipe 11.

Between the inner wall of the large-diameter pipe 12 and the pipe end part 11b of the small-diameter pipe 11, a clearance $\delta_1$ forming part of the second flow channel 12a is provided. The clearance $\delta_1$ is suitably uniform in a fluid flow direction. The clearance $\delta_1$ is suitably 0.02 mm or larger, more suitably 0.05 mm or larger, even more suitably 0.1 mm or larger and is suitably 12.5 mm or smaller, more suitably 6 mm or smaller, even more suitably 1 mm or smaller, and still even more suitably 0.40 mm or smaller. The clearance $\delta_1$ is suitably 0.02 mm or larger and 12.5 mm or smaller, more suitably 0.05 mm or larger and 6 mm or smaller, even more suitably 0.1 mm or larger and 1 mm or smaller, and still even more suitably 0.1 mm or larger and 0.4 mm or smaller.

The fluid merging-contracting part 20 includes a fluid merging part 21 located downstream of the pipe end of the small-diameter pipe 11, and a small hole 22 bored to be continuous with the fluid merging part 21. Consequently, in the fluid merging part 21, the first liquid that has flowed through the first flow channel 11a and the second liquid that has flowed through the second flow channel 12a merge together immediately before the small hole 22, and the resultant fluid immediately after the merging passes through the small hole 22 as it is.

As illustrated in FIG. 2A, the fluid merging part 21 is suitably formed in a tapered cone shape converging toward the small hole 22. This cone convergence angle $\theta_{11}$ is suitably 90° or greater and more suitably 100° or greater and is suitably 180° or less, more suitably 170° or less, and even more suitably 140° or less. The cone convergence angle $\theta_{11}$

is suitably 90° or greater and 180° or less, more suitably 100° or greater and 170° or less, and even more suitably 100° or greater and 140° or less. When a flow channel expanded part 31 has a cone shape, this cone convergence angle $\theta_{11}$ is suitably the same as the cone expansion angle $\theta_{12}$ of the flow channel expanded part 31.

A length $L_1$ from the pipe end of the small-diameter pipe 11 at the fluid merging part 21, that is, from the terminal of the fluid flow channel part 10 to the small hole 22 is suitably 0.02 mm or greater, more suitably 0.2 mm or greater, and even more suitably 0.3 mm or greater and is suitably 20 mm or less, more suitably 10 mm or less, and even more suitably 1 mm or less. This length $L_1$ is suitably 0.02 mm or greater and 20 mm or less, more suitably 0.2 mm or greater and 10 mm or less, and even more suitably 0.3 mm or greater and 1 mm or less.

The cross-sectional shape, the extending direction, the hole diameter $d_1$, the depth $l_1$, the ratio of the depth $l_1$ to the hole diameter $d_1$ (the depth $l_1$/the hole diameter $d_1$), and the flow channel area $s_1$ of the small hole 22 are as described above.

The fluid outflow part 30 includes the flow channel expanded part 31 located downstream of the small hole 22. The flow channel expanded part 31 receives the fluid that has passed through, and flows out of, the small hole 22. The flow channel expanded part 31 communicates with a fluid discharge part 103 provided on the other end of the apparatus.

The cross-sectional shape of the flow channel, the shape, the cone expansion angle $\theta_{12}$ of the fluid outflow part 30, and the maximum flow channel diameter $D_{14}$ of the flow channel expanded part 31 are as described above.

The micromixer 100 of the first configuration may include a plurality of members each of which is formed of metal, ceramics, resin, or the like, and a combination of these members may form the fluid flow channel part 10, the fluid merging-contracting part 20, and the fluid outflow part 30.

In the micromixer 100 of the first configuration, a first liquid supply pipe 42a extending from a first liquid storage tank 41a is connected to the first liquid supply part 101 communicating with the first flow channel 11a. The first liquid supply pipe 42a is provided with a first pump 43a for causing the first liquid to flow, a first flowmeter 44a for detecting a flow rate of the first liquid, and a first filter 45a for removing impurities from in the first liquid in this order from the upstream side. A first pressure gauge 46a for detecting a pressure of the first liquid is attached between the first flowmeter 44a and the first filter 45a. The first pump 43a, the first flowmeter 44a, and the first pressure gauge 46a are each electrically connected to a flow rate controller 47.

A second liquid supply pipe 42b extending from a second liquid storage tank 41b is connected to the second liquid supply part 102 communicating with the second flow channel 12a. The second liquid supply pipe 42b is provided with a second pump 43b for causing the second liquid to flow, a second flowmeter 44b for detecting a flow rate of the second liquid, and a second filter 45b for removing impurities from the second liquid in this order from the upstream side. A second pressure gauge 46b for detecting a pressure of the second liquid is attached between the second flowmeter 44b and the second filter 45b. The second pump 43b, the second flowmeter 44b, and the second pressure gauge 46b are each electrically connected to the flow rate controller 47. In the micromixer system S, since a pressure of the fluid before the passing through the small hole 22 corresponds to a pressure measured by the second pressure gauge 46b, and the fluid after the passing through the small hole 22 flows into an open system and accordingly has a pressure of zero, the pressure loss $\Delta P$ between a pressure upstream of the small hole 22 and a pressure downstream of the small hole 22 is the pressure value measured by the second pressure gauge 46b.

The flow rate controller 47 is capable of receiving input of a set flow rate and a set pressure of the first liquid, incorporates therein an arithmetic element, and controls the operation of the first pump 43a based on set flow rate information of the first liquid, flow rate information detected by the first flowmeter 44a, and pressure information detected by the first pressure gauge 46a. Similarly, the flow rate controller 47 is capable of receiving input of a set flow rate and a set pressure of the second liquid, and controls the operation of the second pump 43b based on set flow rate information of the second liquid, flow rate information detected by the second flowmeter 44b, and pressure information detected by the second pressure gauge 46b.

A fluid collection pipe 48 extends from the fluid discharge part 103 communicating with the flow channel expanded part 31, and is connected to a fluid collection tank 49. A fluid cooler such as a heat exchanger may be provided in the fluid collection pipe 48 or between the fluid collection pipe 48 and the fluid collection tank 49.

The micromixer system S having the configuration as describe above brings the first and second liquids into contact with each other and passes them through the small hole 22 in the micromixer 100 to produce a fluid containing the first and second liquids mixed together through continuous operation. An operation of the micromixer system S will be described next.

After the first liquid and the second liquid are charged into the first liquid storage tank 41a and the second liquid storage tank 41b, the micro mixer system S is actuated. The first pump 43a continuously supplies the first liquid from the first liquid storage tank 41a via the first liquid supply pipe 42a, in which the first liquid passes through the first flowmeter 44a and the first filter 45a in this order, to the first flow channel 11a in the small-diameter pipe 11 through the first liquid supply part 101 of the fluid flow channel part 10. The first flowmeter 44a detects the flow rate information of the first liquid and sends the information to the flow rate controller 47. The first pressure gauge 46a detects the pressure information of the first liquid and sends the information to the flow rate controller 47.

The second pump 43b continuously supplies the second liquid from the second liquid storage tank 41b via the second liquid supply pipe 42b, in which the second liquid passes through the second flowmeter 44b and the second filter 45b in this order, to the second flow channel 12a between the large-diameter pipe 12 and the small-diameter pipe 11 through the second liquid supply part 102 of the fluid flow channel part 10. The second flowmeter 44b detects the flow rate information of the second liquid and sends the information to the flow rate controller 47. The second pressure gauge 46b detects the pressure information of the second liquid and sends the information to the flow rate controller 47.

The flow rate controller 47 controls the operation of the first pump 43a such that the set flow rate and the set pressure of the first liquid are maintained based on the set flow rate information and the set pressure information of the first liquid, the flow rate information being detected by the first flowmeter 44a, and the pressure information being detected by the first pressure gauge 46a. Concurrently therewith, the flow rate controller 47 controls the operation of the second pump 43b such that the set flow rate and the set pressure of the second liquid are maintained based on the set flow rate information and the set pressure information of the second liquid, the flow rate information being detected by the second flowmeter 44b, and the pressure information being detected by the second pressure gauge 46b.

In the fluid flow channel part 10 of the micromixer 100 of the first configuration, the first liquid passes through the first flow channel 11a while the second liquid passes through the second flow channel 12a.

In the fluid merging-contracting part 20, the first and second liquids that have flowed out of the fluid flow channel part 10 are merged together in the fluid merging part 21 in such a manner that the second liquid collides with the flowing first liquid obliquely in the flow direction of the first liquid while the second liquid comes into contact with the entire periphery of the first liquid. In the fluid merging part 21, the fluid formed by merging the first and second liquids together is mixed in a process of passing through the small hole 22.

In the fluid outflow part 30, the fluid that has passed through the small hole 22 flows out to the flow channel expanded part 31. In this fluid, one of the first and second liquids is dispersed in the other. From the fluid discharge part 103 communicating with the flow channel expanded part 31, the obtained fluid is collected to the fluid collection tank 49 via the fluid collection pipe 48.

FIG. 3 illustrates a micromixer 100 of a second configuration. Parts with the same names as those of the micromixer 100 of the first configuration are denoted by the same reference characters as those of the first configuration.

In the micromixer 100 of the second configuration, the pipe end part 11b located in a downstream portion of the small-diameter pipe 11 is bent inwardly over the entire periphery thereof so as to narrow the first flow channel 11a. At the center of the pipe end part 11b, a first liquid outflow hole 13 is provided which has an inner diameter $D_{15}$ smaller than the inner diameter $D_{12}$ of the first flow channel 11a.

The inner diameter $D_{15}$ of the first liquid outflow hole 13 is suitably 0.4 mm or larger and more suitably 1 mm or larger and is suitably 15 mm or smaller, more suitably 12 mm or smaller, and even more suitably 10 mm or smaller. The inner diameter $D_{15}$ is suitably 0.4 mm or leger and 15 mm or smaller, more suitably 1 mm or larger and 12 mm or smaller, and even more suitably 1 mm or larger and 10 mm or smaller.

The outer peripheral portion of the pipe end part 11b has a curved surface swelling outwardly. The inner wall of the large-diameter pipe 12 includes a part corresponding to the pipe end part 11b and having a curved surface recessed inwardly. Further, a portion continuing from the curved surface forms a vertical wall facing the first liquid outflow hole 13. At the center of this portion, the small hole 22 is bored coaxially with the first liquid outflow hole 13. The fluid merging part 21 is formed between the first liquid outflow hole 13 and the small hole 22.

A portion, of the second flow channel 12a, located between the inner wall of the large-diameter pipe 12 and the pipe end part 11b of the small-diameter pipe 11 becomes narrower toward the tip of the pipe end part 11b, and its opening end constitutes a narrowest clearance $\delta_1$. This opening end opens in a direction perpendicular to the opening of the first liquid outflow hole 13. Consequently, the clearance $\delta_1$ also corresponds to the length $L_1$ from the terminal of the fluid flow channel part 10 to the small hole 22. In the fluid merging-contracting part 20, the first and second liquids that have flowed out of the fluid flow channel part 10 merge together in the fluid merging part 21 in such that the second liquid collides with the flowing first liquid in a direction perpendicular to the flow direction of the first liquid while the second liquid comes into contact with the entire periphery of the first liquid. The remaining feature of the second configuration is the same as in the first configuration.

In respect of the embodiment described above, the following configurations will further be disclosed.

<1> A method for producing a ceramide microparticle dispersion, the method including a step of: merging a first mixture in a liquid state containing a ceramide and sterols and a second mixture in a liquid state containing water together, the first and second mixtures having been caused to flow separately from each other; and passing a fluid formed by merging the first and second mixtures together at the step of merging through a small hole having a hole diameter of 0.03 mm or larger and 20 mm or smaller, wherein a mass ratio of a content of a water-soluble organic solvent to a content of the ceramide in the first mixture is 0 or more and 30 or less.

<2> The method according to <1>, in which the content of the ceramide in the first mixture is suitably 0.1% by mass or more, more suitably 1% by mass or more, even more suitably 5% by mass or more, and still even more suitably

10% by mass or more and is suitably 30% by mass or less, more suitably 28% by mass or less, even more suitably 25% by mass or less, and still even more suitably 22% by mass or less.

<3> The method according to <1> or <2>, in which a content of the ceramide in the ceramide microparticle dispersion is suitably 0.1% by mass or more, more suitably 0.3% by mass or more, and even more suitably 0.5% by mass or more and is suitably 10% by mass or less, more suitably 5% by mass or less, and even more suitably 3% by mass or less.

<4> The method according to any one of <1> to <3>, in which the sterols include one or two or more selected from the group consisting of cholesterol, phytosterol, dihydrocholesterol, cholesteryl stearate, cholesteryl nonanoate, cholesteryl hydroxystearate, dihydrocholesteryl oleate, and derivatives and analogues thereof.

<5> The method according to any one of <1> to <4>, in which the sterols include cholesterol.

<6> The method according to any one of <1> to <5>, in which a content of the sterols in the first mixture is suitably 0.5% by mass or more, more suitably 1% by mass or more, and even more suitably 2% by mass or more and is suitably 30% by mass or less, more suitably 20% by mass or less, and even more suitably 15% by mass or less.

<7> The method according to any one of <1> to <6>, in which a content of the sterols in the ceramide microparticle dispersion is suitably 0.1% by mass or more, more suitably 0.2% by mass or more, and even more suitably 0.3% by mass or more and is suitably 5% by mass or less, more suitably 3% by mass or less, and even more suitably 1.5% by mass or less.

<8> The method according to any one of <1> to <7>, in which in the first mixture and/or the ceramide microparticle dispersion, the content of the sterols is the same as the content of the ceramide or less than the content of the ceramide.

<9> The method according to any one of <1> to <8>, in which in the first mixture and/or the ceramide microparticle dispersion, a mass ratio of the content of the sterols to the content of the ceramide is suitably 0.10 or more, more suitably 0.20 or more, and even more suitably 0.30 or more and is suitably 5.0 or less, more suitably 3.0 or less, and even more suitably 1.5 or less.

<10> The method according to any one of <1> to <9>, in which the first mixture contains a water-soluble organic solvent.

<11> The method according to <10>, in which the water-soluble organic solvent includes one or two or more selected from the group consisting of ethanol, propanol, isopropanol, butanol, ethylene glycol, 1,3-propanediol, dipropylene glycol, polybutylene glycol, glycerin, methyl acetate, and ethyl acetate.

<12> The method according to <10> or <11>, in which a content of the water-soluble organic solvent in the first mixture is suitably 0% by mass or more, more suitably 60% by mass or more, and even more suitably 65% by mass or more and is suitably 99% by mass or less, more suitably 95% by mass or less, and even more suitably 90% or less.

<13> The method according to any one of <10> to <12>, in which a content of the water-soluble organic solvent in the ceramide microparticle dispersion is suitably 1% by mass or more, more suitably 3% by mass or more, and even more suitably 5% by mass or more and is suitably 40% by mass or less, more suitably 20% by mass or less, and even more suitably 15% by mass or less.

<14> The method according to any one of <10> to <13>, in which in the first mixture and/or the ceramide microparticle dispersion, a mass ratio of the content of the water-soluble organic solvent to the content of the ceramide is 1.0 or more.

<15> The method according to any one of <10> to <13>, in which in the first mixture and/or the ceramide microparticle dispersion, the mass ratio of the content of the water-soluble organic solvent to the content of the ceramide is 2.0 or more.

<16> The method according to any one of <10> to <13>, in which in the first mixture and/or the ceramide microparticle dispersion, the mass ratio of the content of the water-soluble organic solvent to the content of the ceramide is 3.0 or more.

<17> The method according to any one of <10> to <16>, in which in the first mixture and/or the ceramide microparticle dispersion, the mass ratio of the content of the water-soluble organic solvent to the content of the ceramide is 30 or less.

<18> The method according to any one of <10> to <16>, in which in the first mixture and/or the ceramide microparticle dispersion, the mass ratio of the content of the water-soluble organic solvent to the content of the ceramide is 20 or less.

<19> The method according to any one of <10> to <16>, in which in the first mixture and/or the ceramide microparticle dispersion, the mass ratio of the content of the water-soluble organic solvent to the content of the ceramide is 10 or less.

<20> The method according to any one of <1> to <19>, in which the first mixture contains a surfactant having a neutralizable functional group.

<21> The method according to <20>, in which a content of the surfactant having a neutralizable functional group in the first mixture is suitably 0.5% by mass or more, more suitably 1% by mass or more, and even more suitably 1.5%

by mass or more and is suitably 20% by mass or less, more suitably 15% by mass or less, and even more suitably 10% by mass or less.

<22> The method according to <20> or <21>, in which a content of the surfactant having a neutralizable functional group in the ceramide microparticle dispersion is suitably 0.05% by mass or more, more suitably 0.07% by mass or more, and even more suitably 0.1% by mass or more and is suitably 2% by mass or less, more suitably 1% by mass or less, and even more suitably 0.5% by mass or less.

<23> The method according to any one of <20> to <22>, in which in the first mixture and/or the ceramide microparticle dispersion, a mass ratio of the content of the surfactant having a neutralizable functional group to the content of the ceramide is suitably 0.050 or more, more suitably 0.070 or more, and even more suitably 0.10 or more and is suitably 2.0 or less, more suitably 1.0 or less, and even more suitably 0.50 or less.

<24> The method according to any one of <20> to <23>, in which in the first mixture and/or the ceramide microparticle dispersion, a mass ratio of the content of the surfactant having a neutralizable functional group to the content of the sterols is suitably 0.10 or more, more suitably 0.50 or more, and even more suitably 0.80 or more and is suitably 3.0 or less, more suitably 2.0 or less, and even more suitably 1.5 or less.

<25> The method according to any one of <20> to <24>, in which the second mixture contains a neutralizer having capability of forming a salt with the surfactant having a neutralizable functional group.

<26> The method according to <25>, in which a content of the neutralizer in the second mixture is suitably 0.05% by mass or more, more suitably 0.08% by mass or more, and even more suitably 0.1% by mass or more and is suitably 1.0% by mass or less, more suitably 0.5% by mass or less, and even more suitably 0.2% by mass or less.

<27> The method according to <25> or <26>, in which a content of the neutralizer in the ceramide microparticle dispersion is suitably 0.03% by mass or more, more suitably 0.05% by mass or more, and even more suitably 0.08% by mass or more and is suitably 1.0% by mass or less, more suitably 0.5% by mass or less, and even more suitably 0.2% by mass or less.

<28> The method according to any one of <1> to <27>, in which the first mixture contains a nonionic surfactant.

<29> The method according to <28>, in which an HLB of the nonionic surfactant is suitably 2 or more, more suitably 3 or more, and even more suitably 4 or more and is suitably 15 or less, more suitably 14 or less, and even more suitably 12 or less.

<30> The method according to <28> or <29>, in which a content of the nonionic surfactant in the first mixture is suitably 1% by mass or more, more suitably 3% by mass or more, and even more suitably 5% by mass or more and is suitably 20% by mass or less, more suitably 15% by mass or less, and even more suitably 10% by mass or less.

<31> The method according to any one of <28> to <30>, in which a content of the nonionic surfactant in the ceramide microparticle dispersion is suitably 0.1% by mass or more, more suitably 0.2% by mass or more, and even more suitably 0.4% by mass or more and suitably 2.0% by mass or less, more suitably 1.0% by mass or less, and even more suitably 0.8% by mass or less.

<32> The method according to any one of <28> to <31>, in which in the first mixture and/or the ceramide microparticle dispersion, a mass ratio of the content of the nonionic surfactant to the content of the ceramide is suitably 0.10 or more, more suitably 0.50 or more, and even more suitably 1.0 or more and is suitably 10 or less, more suitably 5.0 or less, and even more suitably 3.0 or less.

<33> The method according to any one of <28> to <32>, in which in the first mixture and/or the ceramide microparticle dispersion, a mass ratio of the content of the surfactant having a neutralizable functional group to the content of the sterols is 0.050 or more.

<34> The method according to any one of <28> to <32>, in which in the first mixture and/or the ceramide microparticle dispersion, the mass ratio of the content of the surfactant having a neutralizable functional group to the content of the sterols is 0.070 or more.

<35> The method according to any one of <28> to <32>, in which in the first mixture and/or the ceramide microparticle dispersion, the mass ratio of the content of the surfactant having a neutralizable functional group to the content of the sterols is 0.10 or more.

<36> The method according to any one of <28> to <35>, in which in the first mixture and/or the ceramide microparticle dispersion, the mass ratio of the content of the surfactant having a neutralizable functional group to the content of the sterols is 3.0 or less.

<37> The method according to any one of <28> to <35>, in which in the first mixture and/or the ceramide microparticle dispersion, the mass ratio of the content of the surfactant having a neutralizable functional group to the content of the sterols is 2.0 or less.

<38> The method according to any one of <28> to <35>, in which in the first mixture and/or the ceramide microparticle dispersion, the mass ratio of the content of the surfactant having a neutralizable functional group to the content of the sterols is 1.0 or less.

<39> The method according to any one of <1> to <38>, in which the first mixture contains both the surfactant having a neutralizable functional group and the nonionic surfactant.

<40> The method according to <39>, in which the sum of the contents of the surfactant having a neutralizable functional group and the nonionic surfactant in the first mixture is suitably 3% by mass or more, more suitably 5% by mass or more, and even more suitably 7% by mass or more and is suitably 25% by mass or less, more suitably 18% by mass or less, and even more suitably 12% by mass or less.
<41> The method according to <39> or <40>, in which in the first mixture and/or the ceramide microparticle dispersion, the content of the nonionic surfactant is higher than the content of the surfactant having a neutralizable functional group.
<42> The method according to any one of <39> to <41>, in which a sum of the contents of the surfactant having a neutralizable functional group and the nonionic surfactant in the ceramide microparticle dispersion is suitably 0.1% by mass or more, more suitably 0.2% by mass or more, and even more suitably 0.4% by mass or more and is suitably 3.0% by mass or less, more suitably 2.0% by mass or less, and even more suitably 1.0% by mass or less.
<43> The method according to any one of <39> to <42>, in which in the first mixture and/or the ceramide microparticle dispersion, a mass ratio of the content of the nonionic surfactant to the content of the surfactant having a neutralizable functional group is suitably 0.50 or more, more suitably 1.0 or more, and even more suitably 2.0 or more and is suitably 10 or less, more suitably 8.0 or less, and even more suitably 5.0 or less.
<44> The method according to any one of <39> to <43>, in which in the first mixture and/or the ceramide microparticle dispersion, a mass ratio of the sum of the contents of the surfactant having a neutralizable functional group and the nonionic surfactant to the content of the ceramide is suitably 0.15 or more, more suitably 0.27 or more, and even more suitably 0.50 or more and is suitably 4.0 or less, more suitably 2.0 or less, and even more suitably 1.0 or less.
<45> The method according to any one of <39> to <44>, in which in the first mixture and/or the ceramide microparticle dispersion, a mass ratio of the sum of the contents of the surfactant having a neutralizable functional group and the nonionic surfactant to the content of the sterols is suitably 0.50 or more, more suitably 1.0 or more, and even more suitably 2.0 or more and is suitably 10 or less, more suitably 6.0 or less, and even more suitably 4.0 or less.
<46> The method according to any one of <1> to <45>, in which a ratio of a flow rate of the first mixture to a flow rate of the second mixture before being merged at the merging step is suitably 1/99 or more, more suitably 3/97 or more, and even more suitably 5/95 or more and is suitably 30/70 or less, more suitably 25/75 or less, and even more suitably 20/80 or less.
<47> The method according to any one of <1> to <46>, in which a temperature of the first mixture before being merged at the merging step is suitably 60°C or higher, more suitably 70°C or higher, and even more suitably 80°C or higher and is suitably 120°C or lower, more suitably 110°C or lower, and even more suitably 100°C or lower.
<48> The method according to any one of <1> to <47>, in which a temperature of the second mixture before being merged at the merging step is suitably 10°C or higher, more suitably 20°C or higher, and even more suitably 30°C or higher and is suitably 90°C or lower, more suitably 80°C or lower, and even more suitably 70°C or lower.
<49> The method according to any one of <1> to <48>, in which before being merged at the merging step, the first mixture has a higher temperature than the second mixture.
<50> The method according to any one of <1> to <49>, in which a manner (angle and the like) of collision when the first mixture and the second mixture are merged together at the merging step is a manner in which the first and second mixtures are caused to collide head-on with each other so as to be merged together, a manner in which one of the first and second mixtures is caused to collide with the other in a direction perpendicular to the other so as be merged therewith, a manner in which one of the first and second mixtures is caused to collide with the other obliquely in the flow direction of the one so that the first and second mixtures are merged together, a manner in which one of the first and second mixtures is caused to collide with the other obliquely in a direction opposite to the flow direction of the one so that the first and second mixtures are merged together, a manner in which one of the first and second mixtures is caused to flow along, and come into contact with, the other so as to to be merged therewith, or a manner in which one of the first and second mixtures is caused to collide with the other while the one comes into contact with the entire periphery of the other so that the first and second mixtures are merged together.
<51> The method according to any one of <1> to <50>, in which a way of merging the first mixture and the second mixture together at the merging step is a manner in which the first and second mixtures are caused to collide with each other in a plurality of directions each so as to be merged together or a manner in which one of the first and second mixtures is caused to collide with the other in a plurality of directions to be merged therewith.
<52> The method according to any one of <1> to <51>, in which a direction in which the small hole extends is the same as at least one of a flow direction of the first mixture or a flow direction of the second mixture.
<53> The method according to any one of <1> to <52>, in which a hole diameter of the small hole is suitably 0.04 mm or larger, more suitably 0.05 mm or larger, even more suitably 0.1 mm or larger, and still even more suitably 0.15 mm or larger and is suitably 10 mm or smaller, more suitably 7 mm or smaller, even more suitably 3 mm or smaller, and still even more suitably 1 mm or smaller.
<54> The method according to any one of <1> to <52>, in which the hole diameter of the small hole is suitably 2.0 mm or larger, more suitably 4.0 mm or larger, and even more suitably 6.0 mm or larger and is suitably 20 mm or

smaller, more suitably 18 mm or smaller, and even more suitably 16 mm or smaller.

<55> The method according to any one of <1> to <54>, in which a depth of the small hole is suitably 0.05 mm or greater, more suitably 0.1 mm or greater, even more suitably 0.3 mm or greater, still even more suitably 0.4 mm or greater, and yet still even more suitably 0.5 mm or greater and is suitably 30 mm or less, more suitably 17 mm or less, even more suitably 9 mm or less, still even more suitably 3 mm or less, and yet still even more suitably 1 mm or less.

<56> The method according to any one of <1> to <55>, in which a ratio of the depth to the hole diameter of the small hole is suitably 0.10 or more, more suitably 0.50 or more, and even more suitably 1.0 or more and is suitably 30 or less, more suitably 10 or less, and even more suitably 5.0 or less.

<57> The method according to any one of <1> to <56>, in which a flow channel area of the small hole is suitably 0.005 $mm^2$ or larger, more suitably 0.01 $mm^2$ or larger, even more suitably 0.02 $mm^2$ or larger, and still even more suitably 0.05 $mm^2$ or larger and is, in view of the same, suitably 180 $mm^2$ or smaller, more suitably 70 $mm^2$ or smaller, even more suitably 8 $mm^2$ or smaller, and still even more suitably 1 $mm^2$ or smaller.

<58> The method for according to any one of <1> to <57>, in which the method for passing the fluid formed by merging the first and second mixtures together through the small hole at the small hole passing step is a method that merges the first and second mixtures together immediately before the small hole and passes the fluid immediately after the merging as it is through the small hole or a method that merges the first and second mixtures together within the small hole and passes the fluid after the merging continuously through the small hole.

<59> The method according to any one of <1> to <58>, in which an average flow velocity of the fluid passing through the small hole at the small hole passing step is suitably 5 m/s or greater, more suitably 10 m/s or greater, and even more suitably 15 m/s or greater and is suitably 70 m/s or less, more suitably 50 m/s or less, and even more suitably 30 m/s or less.

<60> The method according to any one of <1> to <59>, in which the fluid passes through the small hole in turbulence conditions.

<61> The method according to any one of <1> to <60>, in which a Reynolds number of the fluid passing through the small hole at the small hole passing step is suitably 3,000 or greater, more suitably 5,000 or greater, and even more suitably 8,000 or greater and is suitably 300,000 or less, more suitably 250,000 or less, and even more suitably 200,000 or less.

<62> The method according to any one of <1> to <61>, in which a pressure loss between a pressure upstream of the small hole and a pressure downstream of the small hole at the small hole passing step is suitably 0.050 MPa or greater, more suitably 0.1 MPa or greater, and even more suitably 0.2 MPa or greater and is suitably 5.0 MPa or less, more suitably 2.0 MPa or less, and even more suitably 1.0 MPa or less.

<63> The method according to any one of <1> to <62>, in which the fluid that has passed through the small hole at the small hole passing step is caused to flow out to a flow channel expanded part having an expanded flow channel diameter.

<64> The method according to <63>, in which the flow channel expanded part is formed to expand in a cone shape from the small hole to a portion having a maximum flow channel diameter.

<65> The method according to <64>, in which a cone expansion angle of the cone shape is suitably 80° or greater, more suitably 90° or greater, and even more suitably 100° or greater and is suitably 180° or less, more suitably 170° or less, and even more suitably 140° or less.

<66> The method according to any one of <63> to <65>, in which the maximum flow channel diameter of the flow channel expanded part is suitably two times or more, more suitably three times or more, and even more suitably four times or more the hole diameter of the small hole and is suitably 20 times or less, more suitably 12 times or less, and even more suitably 10 times or less the hole diameter of the small hole.

<67> The method according to any one of <1> to <66>, in which a segregation index of an operation to pass the fluid formed by merging the first and second mixtures together through the small hole is suitably 0.1 or less, more suitably 0.01 or less, and even more suitably 0.004 or less.

<68> The method according to any one of <1> to <67>, in which an average particle diameter of the ceramide microparticles in the ceramide microparticle dispersion is suitably 100 nm or smaller, more suitably 80 nm or smaller, and even more suitably 60 nm or smaller.

<69> The method according to any one of <1> to <68>, in which a heat quantity of fusion of the ceramide microparticles in the ceramide microparticle dispersion in a temperature range of 45°C or higher and 80°C or lower is suitably 0.30 J/g or less, more suitably 0.2 J/g or less, and even more suitably 0.1 J/g or less.

<70> A ceramide microparticle dispersion produced by the method of production according to any one of <1> to <69>.

<71> A ceramide microparticle dispersion containing ceramide microparticles, wherein the heat quantity of fusion of the ceramide microparticles in the temperature range of 45°C or higher and 80°C or lower is 0.30 J/g or less.

<72> The ceramide microparticle dispersion according to <70> or <71>, containing the surfactant having a neutralizable functional group.

<73> The ceramide microparticle dispersion according to any one of <70> to <72>, containing sterols.

<74> The ceramide microparticle dispersion according to any one of <70> to <73>, containing sterols and the surfactant having a neutralizable functional group.

<75> The ceramide microparticle dispersion according to <74>, in which the mass ratio of the content of the surfactant having a neutralizable functional group to the content of the sterols in the ceramide microparticle dispersion is 0.010 or more.

<76> The ceramide microparticle dispersion according to <74>, in which the mass ratio of the content of the surfactant having a neutralizable functional group to the content of the sterols in the ceramide microparticle dispersion is 0.10 or more.

<77> The ceramide microparticle dispersion according to <74>, in which the mass ratio of the content of the surfactant having a neutralizable functional group to the content of the sterols in the ceramide microparticle dispersion is 0.30 or more.

<78> The ceramide microparticle dispersion according to any one of <74> to <77>, in which the mass ratio of the content of the surfactant having a neutralizable functional group to the content of the sterols in the ceramide microparticle dispersion is 5.0 or less.

<79> The ceramide microparticle dispersion according to any one of <74> to <77>, in which the mass ratio of the content of the surfactant having a neutralizable functional group to the content of the sterols in the ceramide microparticle dispersion is 4.0 or less.

<80> The ceramide microparticle dispersion according to any one of <74> to <77>, in which the mass ratio of the content of the surfactant having a neutralizable functional group to the content of the sterols in the ceramide microparticle dispersion is 3.0 or less.

<81> Use of the ceramide microparticle dispersion according to any one of <70> to <80>.

## Examples

(Apparatus for Producing Ceramide Microparticle Dispersion)

Using a micromixer system S having the same configuration as that illustrated in FIG. 1 of the embodiment, experiments for producing the following ceramide microparticle dispersions of Examples 1 to 12 and Comparative Example 1 were conducted.

In Examples 1 to 6 and 10 to 16 and Comparative Example 1, used was the micromixer 100 of the first configuration illustrated in FIGS. 2A and 2B: the outer diameter $D_{11}$ of the small-diameter pipe 11 is 3 mm; the inner diameter $D_{12}$ of the small-diameter pipe 11 is 2 mm; the inner diameter $D_{13}$ of the large-diameter pipe 12 is 4.71 mm; the clearance $\Delta_1$ between the small-diameter pipe 11 and the large-diameter pipe 12 is 0.855 mm; the clearance $\delta_1$ between the small-diameter pipe 11 and the large-diameter pipe 12 is 0.25 mm; the length $L_1$ from the terminal of the fluid flow channel part 10 to the small hole 22 is 0.68 mm; the cone diverging angle $\theta_{11}$ of the fluid merging part 21 is 120°; the hole diameter $d_1$ of the small hole 22 is 0.40 mm; the depth $l_1$ of the small hole 22 is 0.55 mm; the flow channel area $s_1$ of the small hole 22 is 0.126 $mm^2$; the depth $l_1$ of the small hole 22/the hole diameter $d_1$ of the small hole 22 is 1.4; the cone expansion angle $\theta_{12}$ of the flow channel expanded part 31 is 120°; the maximum flow channel diameter $D_{14}$ of the flow channel expanded part 31 is 2 mm; and the maximum flow channel diameter $D_{14}$/the hole diameter $d_1$ of the small hole 22 is 5.0.

In Example 7, used was a micromixer having the following configuration: the depth $l_1$ of the small hole 22/the hole diameter $d_1$ of the small hole 22 is 1.8; and the maximum flow channel diameter $D_{14}$/the hole diameter $d_1$ of the small hole 22 is 6.7 as a result of changes of the hole diameter $d_1$ of the small hole 22 to 0.30 mm and the flow channel area $s_1$ of the small hole 22 to 0.0707 $mm^2$. In Example 8, used was a micromixer having the following configuration: the depth $l_1$ of the small hole 22/the hole diameter $d_1$ of the small hole 22 is 1.1; and the maximum flow channel diameter $D_{14}$/the hole diameter $d_1$ of the small hole 22 is 5.0 as a result of changes of the hole diameter $d_1$ of the small hole 22 to 0.50 mm and the flow channel area $s_1$ of the small hole 22 to 0.196 $mm^2$.

In Example 9, used was the micromixer 100 of the second configuration illustrated in FIG. 3: the outer diameter $D_{11}$ of the small-diameter pipe 11 is 20 mm; the inner diameter $D_{12}$ of the small-diameter pipe 11 is 13 mm; the inner diameter $D_{15}$ of the first liquid outflow hole 13 is 8.8 mm; the inner diameter $D_{13}$ of the large-diameter pipe 12 is 25 mm; the clearance $\Delta_1$ between the small-diameter pipe 11 and the large-diameter pipe 12 is 2.5 mm; the clearance $\delta_1$ of the open end between the small-diameter pipe 11 and the large-diameter pipe 12 (i.e., the length $L_1$ from the terminal of the fluid flow channel part 10 to the small hole 22) is 0.86 mm; the hole diameter $d_1$ of the small hole 22 is 6.2 mm; the depth $l_1$ of the small hole 22 is 15.5 mm; the flow channel area $s_1$ of the small hole 22 is 30.2 $mm^2$; the depth $l_1$ of the small hole 22/the hole diameter $d_1$ of the small hole 22 is 2.5; the cone expansion angle $\theta_{12}$ of the flow channel expanded part 31 is 120°; the maximum flow channel diameter $D_{14}$ of the flow channel expanded part 31 is 34 mm; and the maximum flow channel diameter $D_{14}$/the hole diameter $d_1$ of the small hole 22 is 5.5.

In addition, using a homomixer (TK Homomixer Mark II 2.5 manufactured by Primix Corporation), an experiment for producing the following ceramide microparticle dispersion of Comparative Example 2 was conducted.

(Materials Used)

Materials used for the experiments for producing the ceramide microparticle dispersions are as follows:

- Ceramide A (N-(hexadecyloxyhydroxypropyl)-N-hydroxyethyl hexadecanamide): Sphingolipid E manufactured by Kao Corporation
- Ceramide B (Type VI natural ceramide): Ceramide 6 manufactured by Evonik
- Cholesterol: Cholesterol JSQI manufactured by Nippon Fine Chemical Co., Ltd.
- Phytosterol: Phytosterol-SKP manufactured by Tama Biochemical Co., Ltd.
- Surfactant A1 (4D-hydroxy sphinganine): Phytosphingosine manufactured by Evonik
- Surfactant A2 (1-(2-hydroxyethylamino)-3-isostearyloxy-2-propanol): HE-ISP manufactured by Kao Corporation
- Surfactant B (polyoxyethylene octyl dodecyl ether): EMALEX OD-25 (HLB: 14) manufactured by Nihon Emulsion Co., Ltd.
- 1,3-Propanediol: Zemea Select Propanediol manufactured by Iwase Cosfa Co., Ltd.
- Dipropylene glycol: DPG-RF manufactured by Adeka Corporation
- PEG/PPG/polybutylene glycol-8/5/3 glycerin: WILBRIDE S-753 manufactured by NOF Corporation
- Neutralizer: L-glutamic acid manufactured by Ajinomoto Co., Inc.
- Glycerin manufactured by Kao Corporation

Note that Surfactant A1 and Surfactant A2 form a salt through an action of the neutralizer to constitute a cationic surfactant.

(Production of Ceramide Microparticle Dispersion)

Experiments for producing the following ceramide microparticle dispersions of Examples 1 to 16 and Comparative Examples 1 and 2 were conducted. The details thereof are listed in Tables 1A to 1C and Tables 2A to 2C.

<Examples 1, 7, and 8>

As the first mixture, mixed together were Ceramide A, the cholesterol, Surfactant A1, Surfactant B, the 1,3-propanediol, the dipropylene glycol, and the PEG/PPG/polybutylene glycol-8/5/3 glycerin, which were charged into the second liquid storage tank 41b and were temperature-adjusted to have a liquid temperature of 90°C. The contents of the respective components in the ceramide microparticle dispersions to be produced were set as follows: Ceramide A: 1.00% by mass, the cholesterol: 0.15% by mass, Surfactant A1: 0.15% by mass, Surfactant B: 0.50% by mass, the 1,3-propanediol: 1.00% by mass, the dipropylene glycol: 3.00% by mass, and the PEG/PPG/polybutylene glycol-8/5/3 glycerin: 1.00% by mass.

As the second mixture, the neutralizer and ion-exchange water were mixed together, were charged into the first liquid storage tank 41a, and were temperature-adjusted to have a liquid temperature of 65°C. The contents of the respective components in the ceramide microparticle dispersions to be produced were set as follows: the neutralizer: 0.10% by mass and ion-exchange water: 93.10% by mass.

The micromixer system S was operated, and the first mixture and the second mixture were mixed together in the micromixer 100 to produce the ceramide microparticle dispersions.

<Examples 2 to 4>

Ceramide microparticle dispersions were produced by performing operations similar to those of Example 1 except that the contents of the cholesterol in the ceramide microparticle dispersions to be produced were set to 0.30% by mass, 0.50% by mass, and 1.00% by mass, respectively, and the contents of ion-exchange water were set to 92.95% by mass, 92.75% by mass, and 92.25% by mass, respectively.

<Examples 5 and 6>

Ceramide microparticle dispersions were produced by performing operations similar to those of Example 2 except that the temperature of the second mixture was set to 35°C and 85°C, respectively.

<Example 9>

As the first mixture, mixed together were Ceramide A, the cholesterol, Surfactant A2, the dipropylene glycol, and the PEG/PPG/polybutylene glycol-8/5/3 glycerin, which were charged into the second liquid storage tank 41b and were temperature-adjusted to have a liquid temperature of 90°C. The contents of the respective components in the ceramide microparticle dispersion to be produced were set as follows: Ceramide A: 1.00% by mass, the cholesterol: 0.40% by mass, Surfactant A2: 0.40% by mass, the dipropylene glycol: 1.50% by mass, and the PEG/PPG/polybutylene glycol-8/5/3 glycerin: 1.50% by mass.

As the second mixture, the neutralizer, the glycerin, and ion-exchange water were mixed together, were charged into the first liquid storage tank 41a, and were temperature-adjusted to have a liquid temperature of 65°C. The contents of the respective components in the ceramide microparticle dispersion to be produced were set as follows: the neutralizer: 0.16% by mass, the glycerin: 10.00% by mass, and ion-exchange water: 85.04% by mass.

The micromixer system S was operated, and the first mixture and the second mixture were mixed together in the micromixer 100 to produce the ceramide microparticle dispersion.

<Examples 10 to 12>

Ceramide microparticle dispersions were produced by performing operations similar to those of Example 1 except that the contents of Surfactant B in the ceramide microparticle dispersions to be produced were set to 0% by mass, 0.20% by mass, and 1.00% by mass, respectively, and the contents of ion-exchange water were set to 93.60% by mass, 93.40% by mass, and 92.60% by mass, respectively.

<Example 13>

As the first mixture, mixed together were Ceramide B, the cholesterol, Surfactant A1, Surfactant B, the 1,3-propanediol, the dipropylene glycol, and the PEG/PPG/polybutylene glycol-8/5/3 glycerin, which were charged into the second liquid storage tank 41b and were temperature-adjusted to have a liquid temperature of 90°C. The contents of the respective components in the ceramide microparticle dispersion to be produced were set as follows: Ceramide B: 1.00% by mass, the cholesterol: 0.30% by mass, Surfactant A1: 0.15% by mass, Surfactant B: 0.50% by mass, the 1,3-propanediol: 1.00% by mass, the dipropylene glycol: 3.00% by mass, and the PEG/PPG/polybutylene glycol-8/5/3 glycerin: 1.00% by mass.

As the second mixture, the neutralizer and ion-exchange water were mixed together, were charged into the first liquid storage tank 41a, and were temperature-adjusted to have a liquid temperature of 65°C. The contents of the respective components in the ceramide microparticle dispersion to be produced were set as follows: the neutralizer: 0.10% by mass and ion-exchange water: 92.95% by mass.

The micromixer system S was operated, and the first mixture and the second mixture were mixed together in the micromixer 100 to produce the ceramide microparticle dispersion.

<Example 14>

A ceramide microparticle dispersion was produced by performing operations similar to those of Example 2 except that the contents of the 1,3-propanediol, the dipropylene glycol, and the PEG/PPG/polybutylene glycol-8/5/3 glycerin in the ceramide microparticle dispersion to be produced were set to 4.00% by mass, 12.00% by mass, and 4.00% by mass, respectively, and the content of ion-exchange water was set to 77.95% by mass.

<Example 15>

A ceramide microparticle dispersion was produced by performing operations similar to those of Example 2 except that the content of Surfactant Al in the ceramide microparticle dispersion to be produced was set to 0.05% by mass, and the content of ion-exchange water was set to 93.00% by mass.

<Example 16>

As the first mixture, mixed together were Ceramide A, the phytosterol, Surfactant A2, the dipropylene glycol, and the PEG/PPG/polybutylene glycol-8/5/3 glycerin, which were charged into the second liquid storage tank 41b and were temperature-adjusted to have a liquid temperature of 80°C. The contents of the respective components in the ceramide microparticle dispersion to be produced were set as follows: Ceramide A: 1.00% by mass, the phytosterol: 0.40% by mass, Surfactant A2: 0.40% by mass, the dipropylene glycol: 1.50% by mass, and the PEG/PPG/polybutylene

glycol-8/5/3 glycerin: 1.50% by mass.

As the second mixture, the neutralizer, the glycerin, and ion-exchange water were mixed together, were charged into the first liquid storage tank 41a, and were temperature-adjusted to have a liquid temperature of 70°C. The contents of the respective components in the ceramide microparticle dispersion to be produced were set as follows: the neutralizer: 0.16% by mass, the glycerin: 10.00% by mass, and ion-exchange water: 85.04% by mass.

The micromixer system S was operated, and the first mixture and the second mixture were mixed together in the micromixer 100 to produce the ceramide microparticle dispersion.

<Comparative Example 1>

A ceramide microparticle dispersion was produced by performing operations similar to those of Example 1 except that the cholesterol was not used.

<Comparative Example 2>

The same first and second mixtures as those of Example 1 were prepared. The first mixture temperature-adjusted to 90°C was charged into the second mixture temperature-adjusted to 65°C, and the resultant mixture was stirred by rotating the stirring vane of the homomixer at a number of revolutions of 4,000 rpm (with a peripheral speed of the stirring vane of 5.24 m/s) for 5 minutes to produce the ceramide microparticle dispersion.

[Table 1A]

| | | | | Examples 1 | 2 | 3 | 4 | 5 | 6 | 7 | 8 |
|---|---|---|---|---|---|---|---|---|---|---|---|
| Ceramide microparticle dispersion % by mass | First mixture | Ceramide A | | 1.00 (14.7) | 1.00 (14.4) | 1.00 (14.0) | 1.00 (13.1) | 1.00 (14.4) | 1.00 (14.4) | 1.00 (14.7) | 1.00 (14.7) |
| | | Ceramide B | | | | | | | | | |
| | | Cholesterol | | 0.15 (2.21) | 0.30 (4.32) | 0.50 (6.99) | 1.00 (13.1) | 0.30 (4.32) | 0.30 (4.32) | 0.15 (2.21) | 0.15 (2.21) |
| | | Phytosterol | | | | | | | | | |
| | | Surfactant A1 | | 0.15 (2.21) | 0.15 (2.16) | 0.15 (2.10) | 0.15 (1.96) | 0.15 (2.16) | 0.15 (2.16) | 0.15 (2.21) | 0.15 (2.21) |
| | | Surfactant A2 | | | | | | | | | |
| | | Surfactant B | | 0.50 (7.35) | 0.50 (7.19) | 0.50 (6.99) | 0.50 (6.54) | 0.50 (7.19) | 0.50 (7.19) | 0.50 (7.35) | 0.50 (7.35) |
| | | Water-soluble organic solvent | 1,3-Propanediol | 1.00 (14.7) | 1.00 (14.4) | 1.00 (14.0) | 1.00 (13.1) | 1.00 (14.4) | 1.00 (14.4) | 1.00 (14.7) | 1.00 (14.7) |
| | | | Dipropylene glycol | 3.00 (44.1) | 3.00 (43.2) | 3.00 (42.0) | 3.00 (39.2) | 3.00 (43.2) | 3.00 (43.2) | 3.00 (44.1) | 3.00 (44.1) |
| | | | PEG/PPG/polybutylene glycol -8/5/3 glycerin | 1.00 (14.7) | 1.00 (14.4) | 1.00 (14.0) | 1.00 (13.1) | 1.00 (14.4) | 1.00 (14.4) | 1.00 (14.7) | 1.00 (14.7) |
| | | Total | | 6.80 (100) | 6.95 (100) | 7.15 (100) | 7.65 (100) | 6.95 (100) | 6.95 (100) | 6.80 (100) | 6.80 (100) |
| | Second mixture | Neutralizer | | 0.10 (0.11) | 0.10 (0.11) | 0.10 (0.11) | 0.10 (0.11) | 0.10 (0.11) | 0.10 (0.11) | 0.10 (0.11) | 0.10 (0.11) |
| | | Glycerin | | | | | | | | | |
| | | Ion-exchange water | | 93.10 (99.9) | 92.95 (99.9) | 92.75 (99.9) | 92.25 (99.9) | 92.95 (99.9) | 92.95 (99.9) | 93.10 (99.9) | 93.10 (99.9) |
| | | Total | | 93.20 (100) | 93.05 (100) | 92.85 (100) | 92.35 (100) | 93.05 (100) | 93.05 (100) | 93.20 (100) | 93.20 (100) |

* Values within parentheses indicate contents (% by mass) in the first mixture or the second mixture.

[Table 1B]

| | | | | Examples | | | | | | | |
|---|---|---|---|---|---|---|---|---|---|---|---|
| | | | | 9 | 10 | 11 | 12 | 13 | 14 | 15 | 16 |
| Ceramide microparticle % by mass | First mixture | Ceramide A | | 1.00 (20.8) | 1.00 (15.9) | 1.00 (15.4) | 1.00 (13.7) | | 1.00 (4.56) | 1.00 (14.5) | 1.00 (20.8) |
| | | Ceramide B | | | | | | 1.00 (14.4) | | | |
| | | Cholesterol | | 0.40 (8.33) | 0.15 (2.38) | 0.15 (2.31) | 0.15 (2.05) | 0.30 (4.32) | 0.30 (1.37) | 0.30 (4.35) | |
| | | Phytosterol | | | | | | | | | 0.40 (8.33) |
| | | Surfactant A1 | | | 0.15 (2.38) | 0.15 (2.31) | 0.15 (2.05) | 0.15 (2.16) | 0.15 (0.68) | 0.05 (0.72) | |
| | | Surfactant A2 | | 0.40 (8.33) | | | | | | | 0.40 (8.33) |
| | | Surfactant B | | | | 0.20 (3.08) | 1.00 (13.7) | 0.50 (7.19) | 0.50 (2.28) | 0.50 (7.24) | |
| | | Water-soluble organic solvent | 1,3-Propanediol | | 1.00 (15.9) | 1.00 (15.4) | 1.00 (13.7) | 1.00 (14.4) | 4.00 (18.2) | 1.00 (14.5) | |
| | | | Dipropylene glycol | 1.50 (31.3) | 3.00 (47.6) | 3.00 (46.2) | 3.00 (41.1) | 3.00 (43.2) | 12.00 (54.7) | 3.0C (43.5) | 1.50 (31.3) |
| | | | PEG/PPG/polybutylene glycol -8/5/3 glycerin | 1.50 (31.3) | 1.00 (15.9) | 1.00 (15.4) | 1.00 (13.7) | 1.00 (14.4) | 4.00 (18.2) | 1.0 (14.5) | 1.50 (31.3) |
| | | Total | | 4.80 (100) | 6.30 (100) | 6.50 (100) | 7.30 (100) | 6.95 (100) | 21.95 (100) | 6.85 (100) | 4.80 (100) |

(continued)

| | | | Examples | | | | | | | |
|---|---|---|---|---|---|---|---|---|---|---|
| | | | 9 | 10 | 11 | 12 | 13 | 14 | 15 | 16 |
| | Second mixture | Neutralizer | 0.16 (0.17) | 0.10 (0.11) | 0.10 (0.11) | 0.10 (0.11) | 0.10 (0.11) | 0.10 (0.13) | 0.10 (0.11) | 0.16 (0.17) |
| | | Glycerin | 10.00 (10.5) | | | | | | | 10.00 (10.5) |
| | | Ion-exchange water | 85.04 (89.3) | 93.60 (99.9) | 93.40 (99.9) | 92.60 (99.9) | 92.95 (99.9) | 77.95 (99.9) | 93.05 (99.9) | 85.04 (89.3) |
| | | Total | 95.20 (100) | 93.70 (100) | 93.50 (100) | 92.70 (100) | 93.05 (100) | 78.05 (100) | 93.15 (100) | 95.20 (100) |

* Values within parentheses indicate contents (% by mass) in the first mixture or the second mixture.

[Table 1C]

| | | | | Comparative Examples 1 | Comparative Examples 2 |
|---|---|---|---|---|---|
| Ceramide Microparticle Dispersion % by mass | First mixture | Ceramide A | | 1.00 (15.0) | 1.00 (14.7) |
| | | Ceramide B | | | |
| | | Cholesterol | | | 0.15 (2.21) |
| | | Phytosterol | | | |
| | | Surfactant A1 | | 0.15 (2.26) | 0.15 (2.21) |
| | | Surfactant A2 | | | |
| | | | Surfactant B | 0.50 (7.52) | 0.50 (7.35) |
| | | Water-soluble organic solvent | 1,3-Propanediol | 1.00 (15.0) | 1.00 (14.7) |
| | | | Dipropylene glycol | 3.00 (45.1) | 3.00 (44.1) |
| | | | PEG/PPG/ polybutylene glycol -8/5/3 glycerin | 1.00 (15.0) | 1.00 (14.7) |
| | | Total | | 6.65 (100) | 6.80 (100) |
| | Second mixture | Neutralizer | | 0.10 (0.11) | 0.10 (0.11) |
| | | Glycerin | | | |
| | | Ion-exchange water | | 93.25 (99.9) | 93.10 (99.9) |
| | | Total | | 93.35 (100) | 93.20 (100) |

* Values within parentheses indicate contents (% by mass) in the first mixture or the second mixture.

[Table 2A]

| | | Examples 1 | 2 | 3 | 4 | 5 | 6 | 7 | 8 |
|---|---|---|---|---|---|---|---|---|---|
| Sterols/Ceramide | | 0.15 | 0.30 | 0.50 | 1.0 | 0.30 | 0.30 | 0.15 | 0.15 |
| Water-soluble organic solvent (% by mass) | Within first mixture | 73.5 | 71.9 | 69.9 | 65.4 | 71.9 | 71.9 | 73.5 | 73.5 |
| | Within dispersion | 5.00 | 5.00 | 5.00 | 5.00 | 5.00 | 5.00 | 5.00 | 5.00 |

(continued)

| | | Examples | | | | | | | |
|---|---|---|---|---|---|---|---|---|---|
| | | 1 | 2 | 3 | 4 | 5 | 6 | 7 | 8 |
| Water-soluble organic solvent/ Ceramide | Within first mixture | 5.0 | 5.0 | 5.0 | 5.0 | 5.0 | 5.0 | 5.0 | 5.0 |
| | Within dispersion | 5.0 | 5.0 | 5.0 | 5.0 | 5.0 | 5.0 | 5.0 | 5.0 |
| Surfactant A/Ceramide | | 0.15 | 0.15 | 0.15 | 0.15 | 0.15 | 0.15 | 0.15 | 0.15 |
| Surfactant A/Sterols | | 1.0 | 0.50 | 0.30 | 0.15 | 0.50 | 0.50 | 1.0 | 1.0 |
| Surfactant B/Ceramide | | 0.50 | 0.50 | 0.50 | 0.50 | 0.50 | 0.50 | 0.50 | 0.50 |
| Surfactant B/Sterols | | 3.3 | 1.7 | 1.0 | 0.50 | 1.7 | 1.7 | 3.3 | 3.3 |
| Surfactant A + Surfactant B (% by mass) | Within first mixture | 9.55 | 9.35 | 9.09 | 8.50 | 9.35 | 9.35 | 9.55 | 9.55 |
| | Within dispersion | 0.65 | 0.65 | 0.65 | 0.65 | 0.65 | 0.65 | 0.65 | 0.65 |
| Surfactant B/Surfactant A | | 3.3 | 3.3 | 3.3 | 3.3 | 3.3 | 3.3 | 3.3 | 3.3 |
| (Surfactant A + Surfactant B) /Ceramide | | 0.65 | 0.65 | 0.65 | 0.65 | 0.65 | 0.65 | 0.65 | 0.65 |
| (Surfactant A + Surfactant B) /Sterols | | 4.3 | 2.2 | 1.3 | 0.65 | 2.2 | 2.2 | 4.3 | 4.3 |
| Flow rate $Q_1$ (kg/h) of first mixture before merging | | 0.696 | 0.708 | 0.732 | 0.780 | 0.708 | 0.708 | 0.816 | 0.408 |
| Flow rate $Q_2$ (kg/h) of second mixture before merging | | 9.50 | 9.49 | 9.47 | 9.42 | 9.49 | 9.49 | 11.2 | 5.59 |
| $Q_1/Q_2$ | | 0.0733 | 0.0746 | 0.0773 | 0.0828 | 0.0746 | 0.0746 | 0.0729 | 0.0730 |
| Flow rate Q (kg/h) of fluid after merging | | 10.2 | 10.2 | 10.2 | 10.2 | 10.2 | 10.2 | 12.0 | 6.00 |
| Temperature (°C) of first mixture before merging | | 90 | 90 | 90 | 90 | 90 | 90 | 90 | 90 |
| Temperature (°C) of second mixture before merging | | 65 | 65 | 65 | 65 | 35 | 85 | 65 | 65 |
| Temperature difference (°C) between first and second mixtures | | 25 | 25 | 25 | 25 | 55 | 5.0 | 25 | 25 |
| Hole diameter (mm) of small hole | | 0.40 | 0.40 | 0.40 | 0.40 | 0.40 | 0.40 | 0.30 | 0.50 |
| Pressure loss $\Delta P$ (MPa) between pressures upstream and downstream of small hole | | 0.40 | 0.40 | 0.40 | 0.40 | 0.40 | 0.40 | 1.7 | 0.080 |
| Average particle diameter (nm) | | 56.9 | 56.9 | 60.7 | 69.3 | 70.1 | 54.9 | 50.5 | 81.1 |
| Heat quantity of fusion (J/g) | | 0.30 | 0.065 | 0.00045 | 0.0010 | 0.10 | 0.060 | 0.34 | 0.38 |

[Table 2B]

| | | Examples | | | | | | | |
|---|---|---|---|---|---|---|---|---|---|
| | | 9 | 10 | 11 | 12 | 13 | 14 | 15 | 16 |
| Sterol s/Ceramide | | 0.40 | 0.15 | 0.15 | 0.15 | 0.30 | 0.30 | 0.30 | 0.40 |
| Water-soluble organic solvent (% by mass) | Within first mixture | 62.5 | 79.4 | 76.9 | 68.5 | 71.9 | 91.1 | 73.0 | 62.5 |
| | Within dispersion | 13.00 | 5.00 | 5.00 | 5.00 | 5.00 | 20.00 | 5.00 | 13.00 |
| Water-soluble organic solvent/ Ceramide | Within first mixture | 3.0 | 5.0 | 5.0 | 5.0 | 5.0 | 20 | 5.0 | 3.0 |
| | Within dispersion | 13 | 5.0 | 5.0 | 5.0 | 5.0 | 20 | 5.0 | 13 |
| Surfactant A/Ceramide | | 0.40 | 0.15 | 0.15 | 0.15 | 0.15 | 0.15 | 0.05 | 0.40 |
| Surfactant A/Sterols | | 1.0 | 1.0 | 1.0 | 1.0 | 0.50 | 0.50 | 0.17 | 1.0 |
| Surfactant B/Ceramide | | - | - | 0.20 | 1.0 | 0.50 | 0.50 | 0.50 | - |
| Surfactant B/Sterols | | - | - | 1.3 | 6.7 | 1.7 | 1.7 | 1.7 | - |
| Surfactant A + Surfactant B (% by mass) | Within first mixture | 8.33 | 2.38 | 5.38 | 15.8 | 9.35 | 2.96 | 8.03 | 8.33 |
| | Within dispersion | 0.40 | 0.15 | 0.35 | 1.2 | 0.65 | 0.65 | 0.55 | 0.40 |
| Surfactant B/Surfactant A | | - | - | 1.3 | 6.7 | 3.3 | 3.3 | 10 | - |
| (Surfactant A + Surfactant B)/ Ceramide | | 0.40 | 0.15 | 0.35 | 1.2 | 0.65 | 0.65 | 0.55 | 0.40 |
| (Surfactant A + Surfactant B)/ Sterols | | 1.0 | 1.0 | 2.3 | 7.7 | 2.2 | 2.2 | 1.8 | 1.0 |
| Flow rate $Q_1$ (kg/h) of first mixture before merging | | 120 | 0.642 | 0.666 | 0.744 | 0.708 | 2.24 | 0.699 | 0.461 |
| Flow rate $Q_2$ (kg/h) of second mixture before merging | | 2370 | 9.56 | 9.54 | 9.46 | 9.49 | 7.96 | 9.50 | 9.14 |
| $Q_1/Q_2$ | | 0.0506 | 0.0672 | 0.0698 | 0.0786 | 0.0746 | 0.281 | 0.0735 | 0.0504 |
| Flow rate Q (kg/h) of fluid after merging | | 2490 | 10.2 | 10.2 | 10.2 | 10.2 | 10.2 | 10.2 | 9.6 |
| Temperature (°C) of first mixture before merging | | 90 | 90 | 90 | 90 | 90 | 90 | 90 | 80 |
| Temperature (°C) of second mixture before merging | | 65 | 65 | 65 | 65 | 65 | 65 | 65 | 70 |
| Temperature difference (°C) between first and second mixtures | | 25 | 25 | 25 | 25 | 25 | 25 | 25 | 10 |
| Hole diameter (mm) of small hole | | 6.2 | 0.40 | 0.40 | 0.40 | 0.40 | 0.40 | 0.40 | 0.40 |
| Pressure loss $\Delta P$ (MPa) between pressures upstream and downstream of small hole | | 0.59 | 0.40 | 0.40 | 0.40 | 0.40 | 0.40 | 0.40 | 0.40 |
| Average particle diameter (nm) | | 77.6 | 160 | 88.7 | 55.7 | 50.6 | 56.5 | 77.3 | 48.1 |

(continued)

| | Examples | | | | | | | |
|---|---|---|---|---|---|---|---|---|
| | 9 | 10 | 11 | 12 | 13 | 14 | 15 | 16 |
| Heat quantity of fusion (J/g) | 0.0093 | 0.084 | 0.26 | 0.35 | 0.0012 | 0.42 | 0.216 | 0.011 |

[Table 2C]

| | | Comparative Examples | |
|---|---|---|---|
| | | 1 | 2 |
| Sterols/Ceramide | | - | 0.15 |
| Water-soluble organic solvent (% by mass) | Within first mixture | 75.2 | 73.5 |
| | Within dispersion | 5.00 | 5.00 |
| Water-soluble organic solvent/Ceramide | Within first mixture | 5.0 | 5.0 |
| | Within dispersion | 5.0 | 5.0 |
| Surfactant A/Ceramide | | 0.15 | 0.15 |
| Surfactant A/Sterols | | - | 1.0 |
| Surfactant B/Ceramide | | 0.50 | 0.50 |
| Surfactant B/Sterols | | - | 3.3 |
| Surfactant A + Surfactant B (% by mass) | Within first mixture | 9.77 | 9.56 |
| | Within dispersion | 0.65 | 0.65 |
| Surfactant B/Surfactant A | | 3.3 | 3.3 |
| (Surfactant A + Surfactant B)/Ceramide | | 0.65 | 0.65 |
| (Surfactant A + Surfactant B)/Sterols | | - | 4.3 |
| Flow rate $Q_1$ (kg/h) of first mixture before merging | | 0.678 | - |
| Flow rate $Q_2$ (kg/h) of second mixture before merging | | 9.52 | - |
| $Q_1/Q_2$ | | 0.0712 | - |
| Flow rate Q (kg/h) of fluid after merging | | 10.2 | - |
| Temperature (°C) of first mixture before merging | | 90 | 90 |
| Temperature (°C) of second mixture before merging | | 65 | 65 |
| Temperature difference (°C) between first and second mixtures | | 25 | 25 |
| Hole diameter (mm) of small hole | | 0.40 | - |
| Pressure loss $\Delta P$ (MPa) between pressures upstream and downstream of small hole | | 0.40 | - |
| Average particle diameter (nm) | | 40.8 | 191 |
| Heat quantity of fusion (J/g) | | 0.76 | 0.38 |

(Method of Testing)

For each of the ceramide microparticle dispersions produced in Examples 1 to 16 and Comparative Examples 1 and 2, a cumulant diameter was measured by photon correlation method (dynamic light scattering) using a particle size distribution measurement apparatus (ELS-Z manufactured by Otsuka Electronics Co., Ltd.). The measured cumulant diameter was determined to be the average particle diameter of the ceramide microparticles.

For each of the ceramide microparticle dispersions produced in Examples 1 to 16 and Comparative Examples 1 and 2, a heat quantity of fusion was determined from a total endotherm in a temperature range of 45°C or higher and

80°C or lower of a melting profile obtained by measurement (temperature raising rate: 0.3°C/min) using a differential scanning calorimetric analyzer (μDSC VII evo manufactured by Rigaku Corporation).

(Test Results)

Tables 2 show test results.

Referring to Tables 2, a comparison between Examples 1 to 16 and Comparative Examples 1 and 2 shows that Examples 1 to 16 are smaller in the average particle diameter and are lower in the heat quantity of fusion than Comparative Examples 1 and 2. This means that Examples 1 to 16 are lower in the crystallinity of the ceramide than Comparative Examples 1 and 2.

<Cholesterol Content>

Regarding Examples 1 to 4 and Comparative Example 1, in which the cholesterol content in the ceramide microparticle dispersions was varied, FIG. 4A shows a relation between the cholesterol content and the average particle diameter of the ceramide microparticles in the ceramide microparticle dispersions. FIG. 4B shows a relation between the cholesterol content and the heat quantity of fusion of the ceramide microparticles in the ceramide microparticle dispersions.

It is revealed from FIG. 4A that as the cholesterol content increases, the average particle diameter of the ceramide microparticles slightly increases. It is revealed from FIG. 4B that as the cholesterol content increases, the heat quantity of fusion of the ceramide microparticles remarkably decreases, and thus the crystallinity thereof decreases until the cholesterol content reaches about 0.50% by mass, and that a higher cholesterol content than about 0.50% by mass makes the heat quantity of fusion be nearly 0 J/g, that is, achieves amorphousness.

<Temperature of Second Mixture>

Regarding Examples 2, 5, and 6, in which the temperature of the second mixture was varied, FIG. 5A shows a relation between the temperature of the second mixture and the average particle diameter of the ceramide microparticles. FIG. 5B shows a relation between the temperature of the second mixture and the heat quantity of fusion of the ceramide microparticles.

It is revealed from FIG. 5A that as the temperature of the second mixture rises, that is, the temperature difference with respect to the first mixture decreases, the average particle diameter of the ceramide microparticles decreases. It is revealed from FIG. 5B that as the temperature of the second mixture rises, the heat quantity of fusion of the ceramide microparticles slightly decreases, and thus the crystallinity thereof decreases.

<Hole Diameter of Small Hole>

Regarding Examples 1, 7, and 8, in which the hole diameter of the small hole was varied, FIG. 6A shows a relation between the hole diameter of the small hole and the average particle diameter of the ceramide microparticles. FIG. 6B shows a relation between the hole diameter of the small hole and the heat quantity of fusion of the ceramide microparticles.

It is revealed from FIG. 6A that as the hole diameter of the small hole increases, the average diameter of the ceramide microparticles increases. It is revealed from FIG. 6B that there is no correlation between the hole diameter of the small hole and the heat quantity of fusion of the ceramide microparticles.

<Content of Surfactant B>

Regarding Examples 1 and 10 to 12, in which the content of Surfactant B in the ceramide microparticle dispersions was varied, FIG. 7A shows a relation between the content of Surfactant B in the ceramide microparticle dispersions and the average particle diameter of the ceramide microparticles. FIG. 7B shows a relation between the content of Surfactant B in the ceramide microparticle dispersions and the heat quantity of fusion of the ceramide microparticles.

It is revealed from FIG. 7A that as the content of Surfactant B increases, the average particle diameter of the ceramide microparticles remarkably decreases until the content of Surfactant B reaches about 0.50% by mass, and that a higher content of Surfactant B than about 0.50% by mass makes the ceramide microparticles have a generally constant average particle diameter slightly smaller than 60 nm. It is revealed from FIG. 7B that as the content of Surfactant B increases, the heat quantity of fusion of the ceramide microparticles sharply increases, and thus the crystallinity thereof increases until the content of Surfactant B reaches about 0.20% by mass, that once the content of Surfactant B exceeds about 0.20% by mass, as the content of Surfactant B increases, the heat quantity of fusion of the ceramide microparticles

gradually increases, and thus the crystallinity thereof increases.

INDUSTRIAL APPLICABILITY

The present invention is useful in the technical fields of a ceramide microparticle dispersion and a method for producing the same.

DESCRIPTION OF REFERENCE CHARACTERS

22 Small Hole

## Claims

**1.** A method for producing a ceramide microparticle dispersion, the method comprising the steps of:

merging a first mixture in a liquid state containing a ceramide and sterols and a second mixture in a liquid state containing water together, the first and second mixtures having been caused to flow separately; and
passing a fluid formed by merging the first and second mixtures together at the step of merging through a small hole having a hole diameter of 0.03 mm or larger and 20 mm or smaller, wherein
a mass ratio of a content of a water-soluble organic solvent to a content of the ceramide in the first mixture is 0 or more and 30 or less.

**2.** The method of claim 1, wherein
the first mixture contains a water-soluble organic solvent.

**3.** The method of claim 1 or 2, wherein
the sterols include cholesterol.

**4.** The method of any one of claims 1 to 3, wherein
a mass ratio of a content of the sterols to the content of the ceramide in the first mixture is 0.20 or more and 1.0 or less.

**5.** The method of any one of claims 1 to 4, wherein
before being merged at the step of merging, the first mixture has a higher temperature than the second mixture.

**6.** The method of any one of claims 1 to 5, wherein
a heat quantity of fusion of ceramide microparticles contained in the ceramide microparticle dispersion in a temperature range of 45°C or higher and 80°C or lower is 0.30 J/g or less.

**7.** The method of any one of claims 1 to 6, wherein
the first mixture contains a surfactant having a neutralizable functional group.

**8.** The method of claim 7, wherein
the second mixture contains a neutralizer having capability of forming a salt with the surfactant having the neutralizable functional group.

**9.** The method of claim 7 or 8, wherein
a mass ratio of a content of the surfactant having the neutralizable functional group to the content of the ceramide in the first mixture is 0.050 or more and 0.20 or less.

**10.** The method of any one of claims 1 to 9, wherein
the first mixture contains a nonionic surfactant.

**11.** The method of claim 10, wherein
a mass ratio of a content of the nonionic surfactant to the content of the ceramide in the first mixture is 0.050 or more and 1.0 or less.

**12.** The method of any one of claims 1 to 11, wherein
an average particle diameter of ceramide microparticles contained in the ceramide microparticle dispersion is 100 nm or smaller.

**13.** A ceramide microparticle dispersion comprising ceramide microparticles, wherein
a heat quantity of fusion of the ceramide microparticles in a temperature range of 45°C or higher and 80°C or lower is 0.30 J/g or less.

**14.** The ceramide microparticle dispersion of claim 13, further comprising a surfactant having a neutralizable functional group.

**15.** The ceramide microparticle dispersion of claim 13 or 14, further comprising sterols.

**16.** The ceramide microparticle dispersion of any one of claims 13 to 15, further containing a surfactant having a neutralizable functional group and sterols, wherein
a mass ratio of a content of the surfactant having the neutralizable functional group to a content of the sterols in the ceramide microparticle dispersion is 0.010 or more and 5.0 or less.

FIG.1

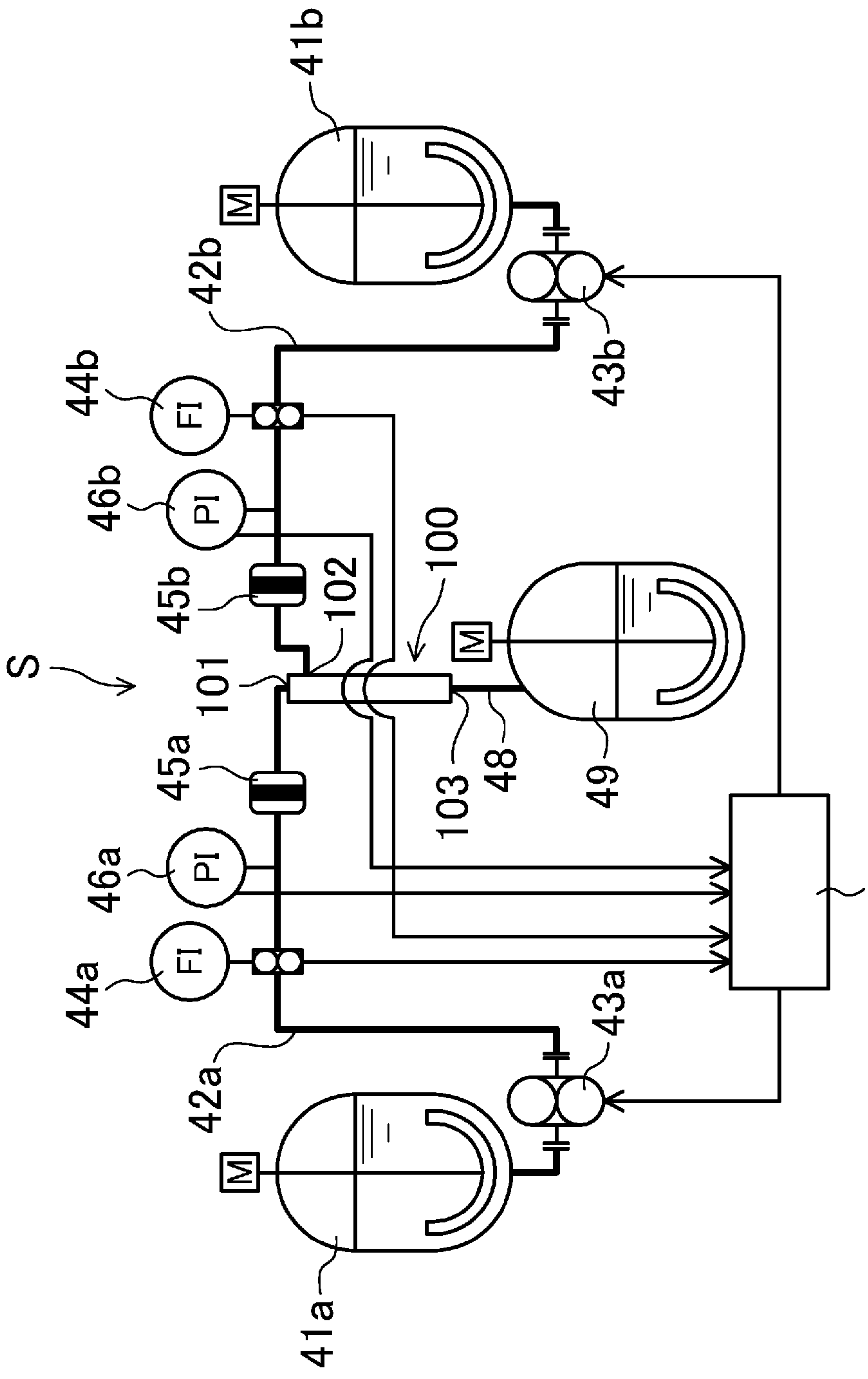
S
41a
41b
42a
42b
43a
43b
44a
44b
45a
45b
46a
46b
47
48
49
100
101
102
103
FI
FI
PI
PI
M
M
M

FIG.2A

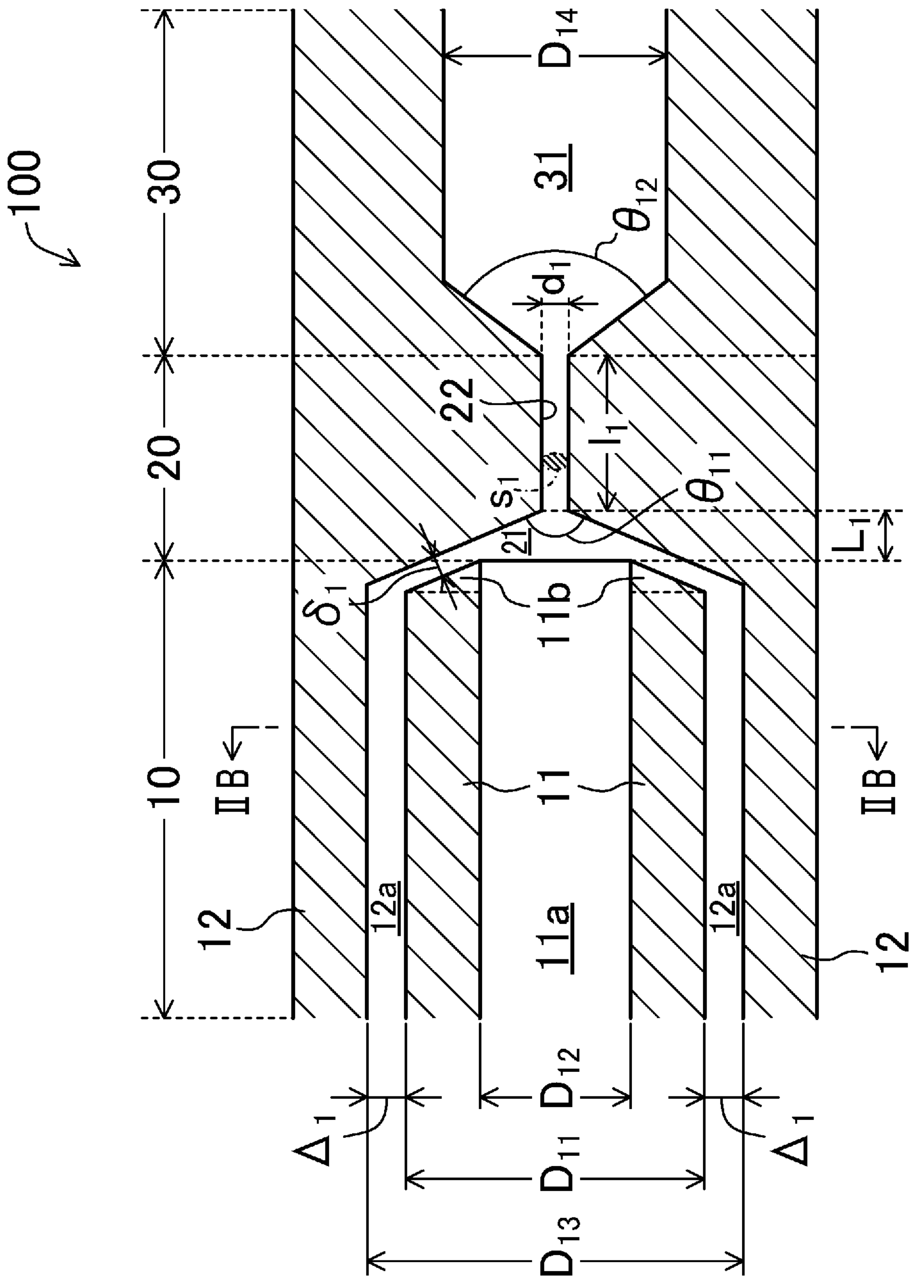
100
10
20
30
$D_{14}$
31
$\theta_{12}$
$d_1$
22
$l_1$
$\theta_{11}$
$s_1$
21
$L_1$
$\delta_1$
11b
11
11a
12
12a
IIB
$D_{12}$
$D_{11}$
$D_{13}$
$\Delta_1$

FIG.2B

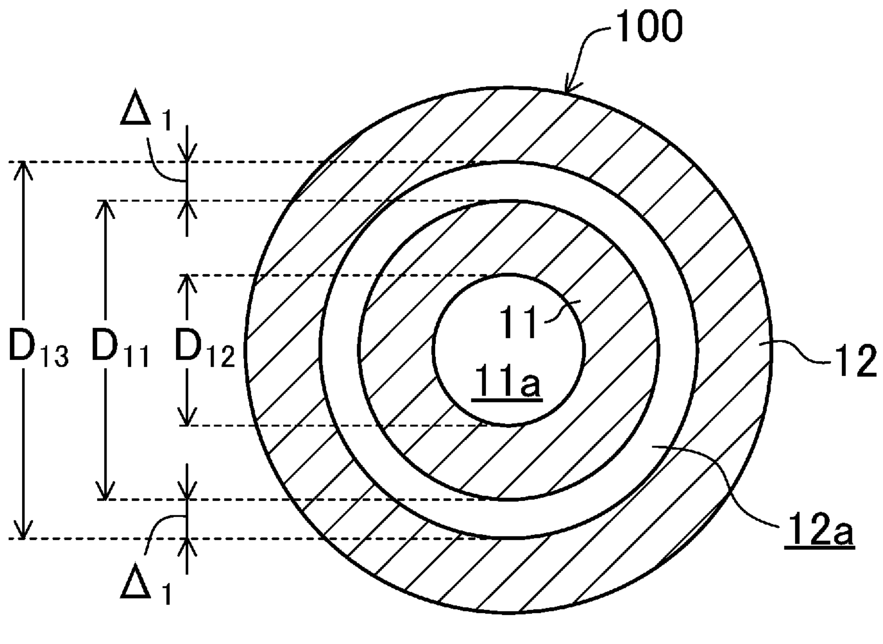

FIG.3

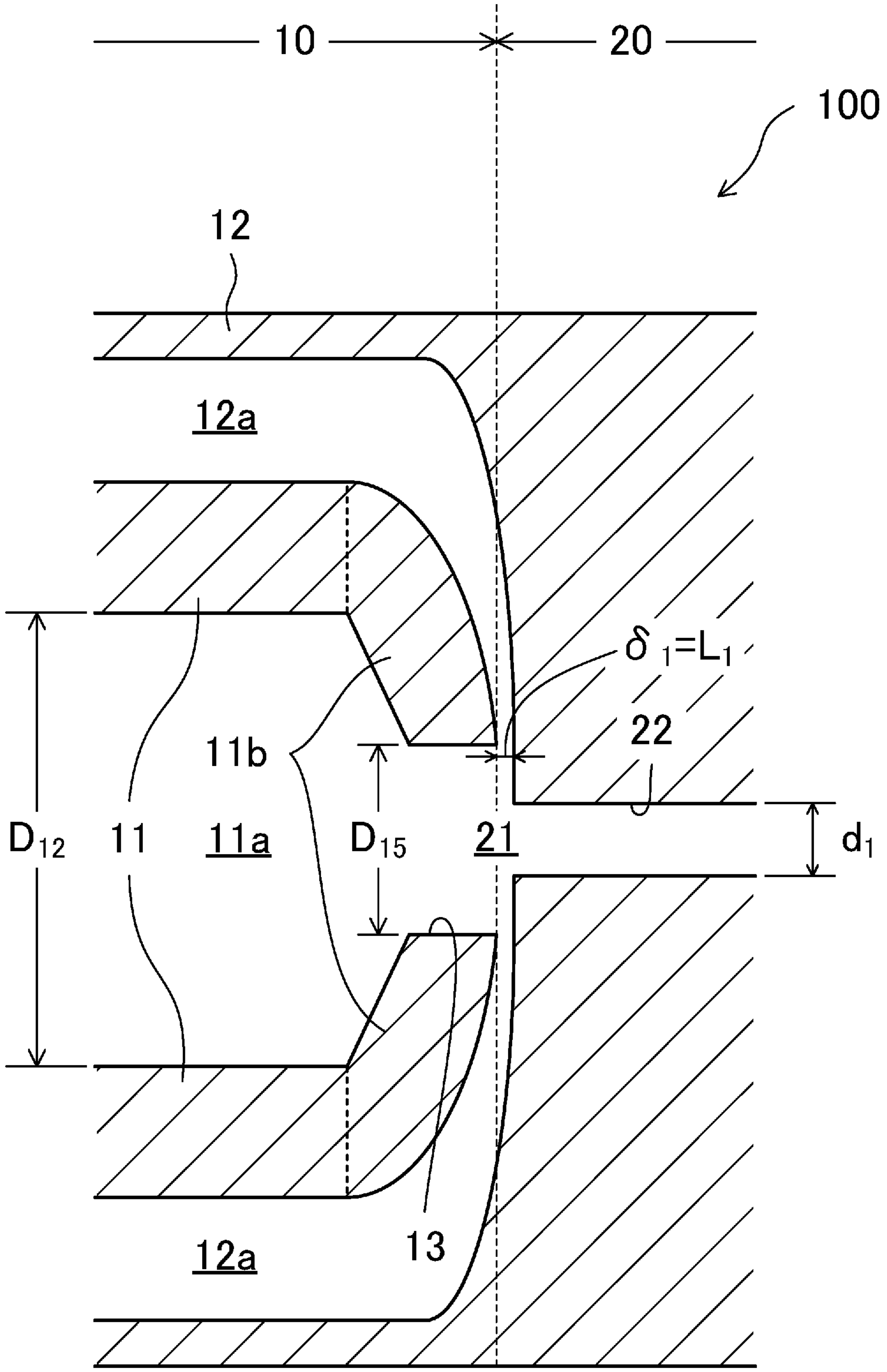

FIG.4A

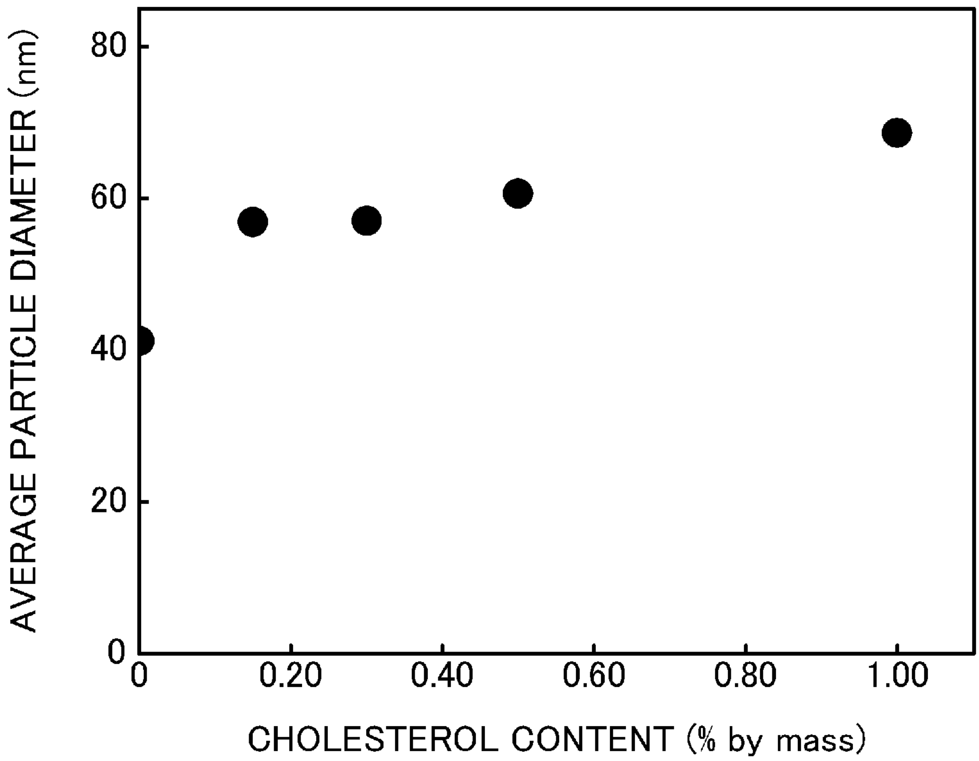
80
60
40
20
0
0
0.20
0.40
0.60
0.80
1.00
AVERAGE PARTICLE DIAMETER (nm)
CHOLESTEROL CONTENT (% by mass)

FIG.4B

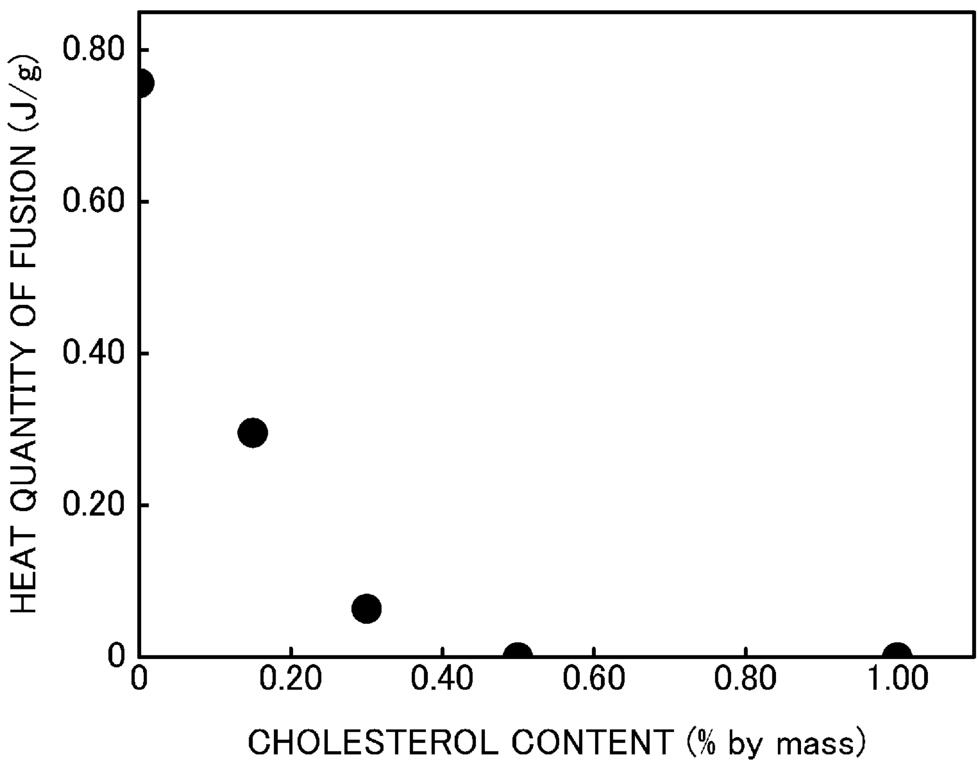
0.80
0.60
0.40
0.20
0
0
0.20
0.40
0.60
0.80
1.00
HEAT QUANTITY OF FUSION (J/g)
CHOLESTEROL CONTENT (% by mass)

FIG.5A

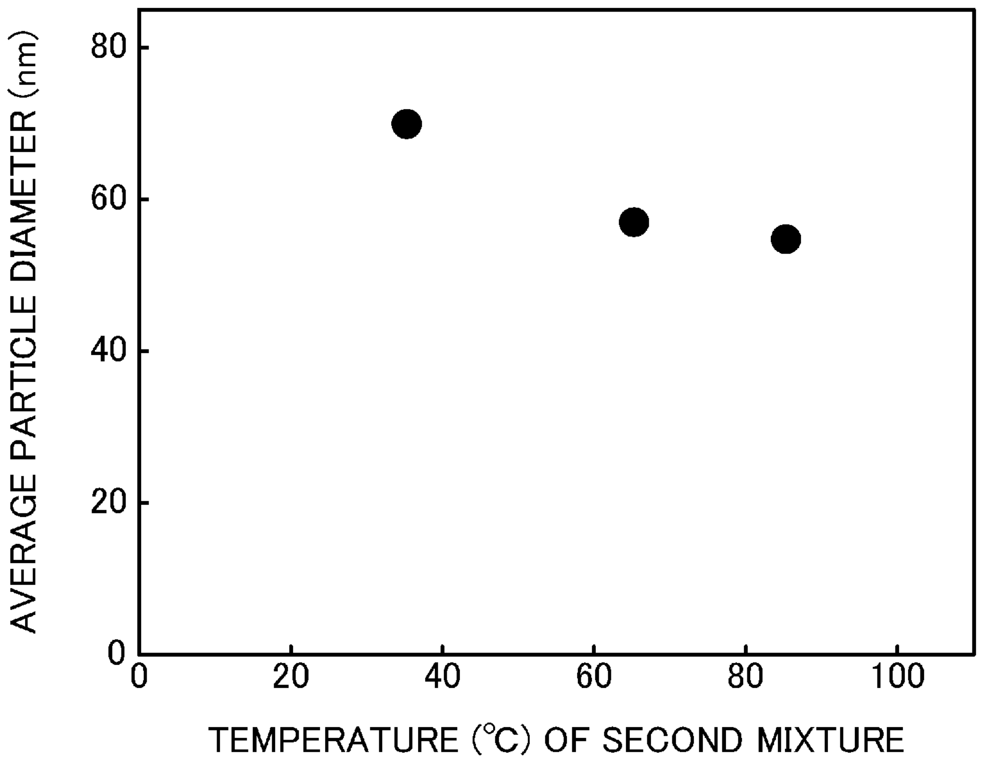
AVERAGE PARTICLE DIAMETER (nm)
80
60
40
20
0
0
20
40
60
80
100
TEMPERATURE (°C) OF SECOND MIXTURE

FIG.5B

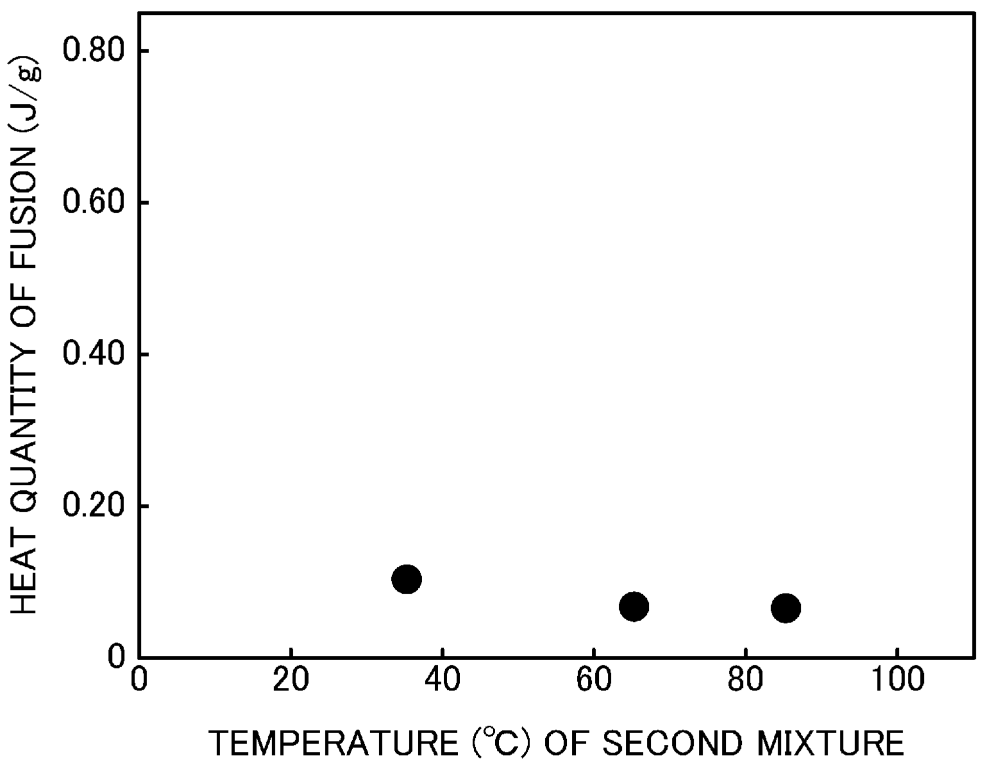
HEAT QUANTITY OF FUSION (J/g)
0.80
0.60
0.40
0.20
0
0
20
40
60
80
100
TEMPERATURE (°C) OF SECOND MIXTURE

FIG.6A

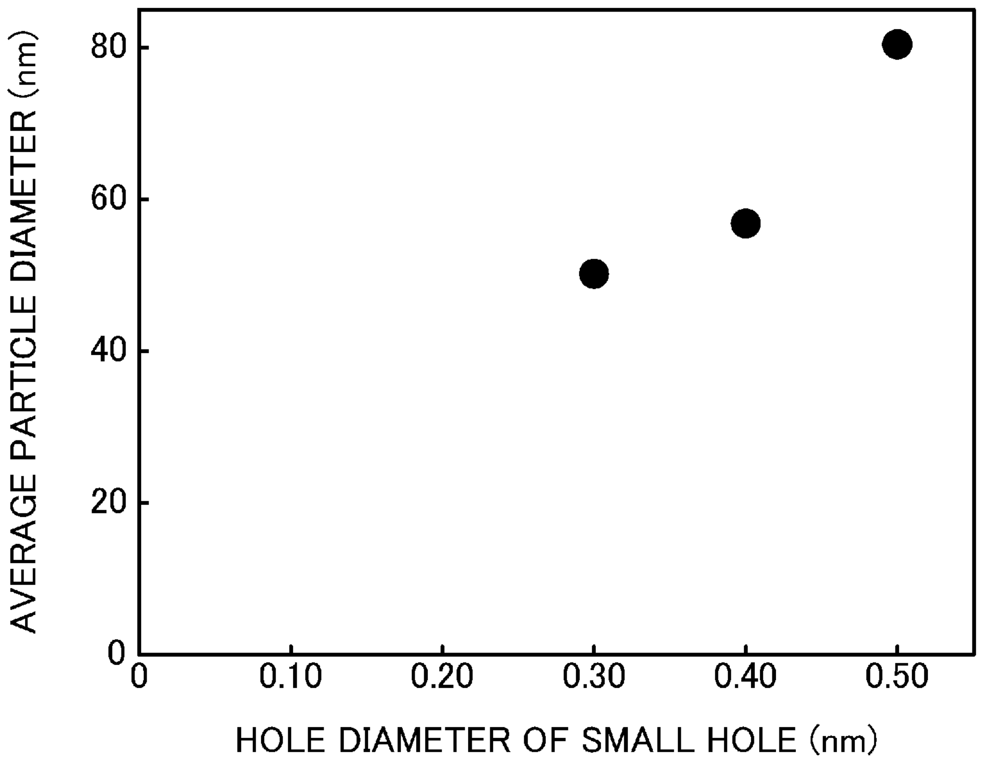
AVERAGE PARTICLE DIAMETER (nm)
80
60
40
20
0
0
0.10
0.20
0.30
0.40
0.50
HOLE DIAMETER OF SMALL HOLE (nm)

FIG.6B

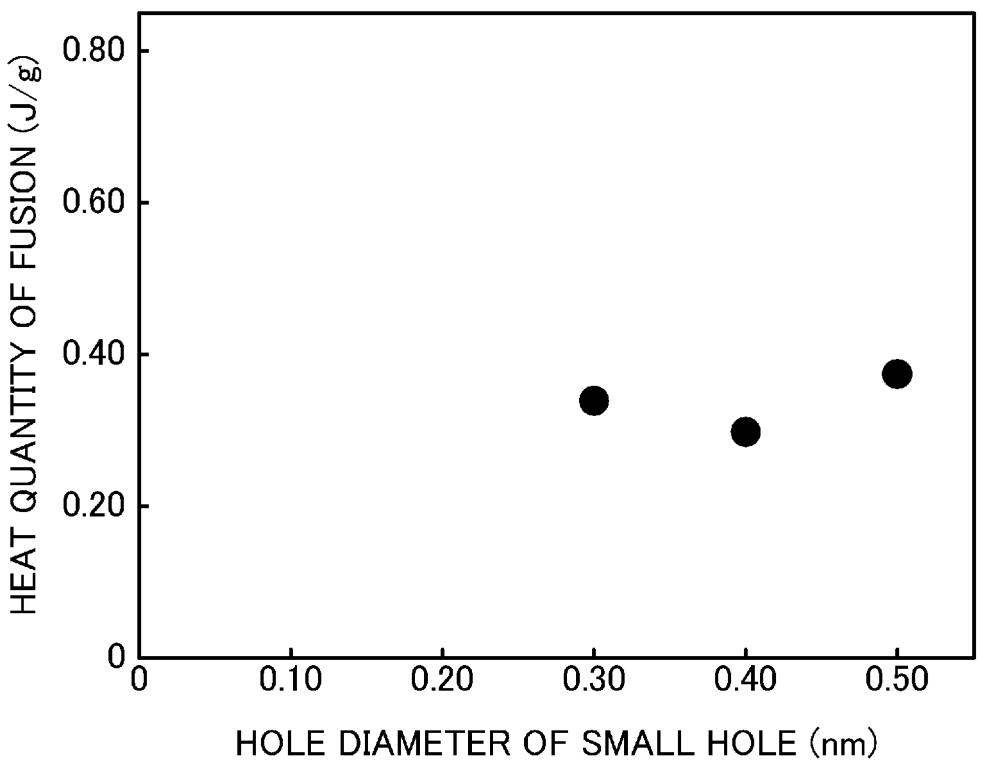
HEAT QUANTITY OF FUSION (J/g)
0.80
0.60
0.40
0.20
0
0
0.10
0.20
0.30
0.40
0.50
HOLE DIAMETER OF SMALL HOLE (nm)

FIG.7A

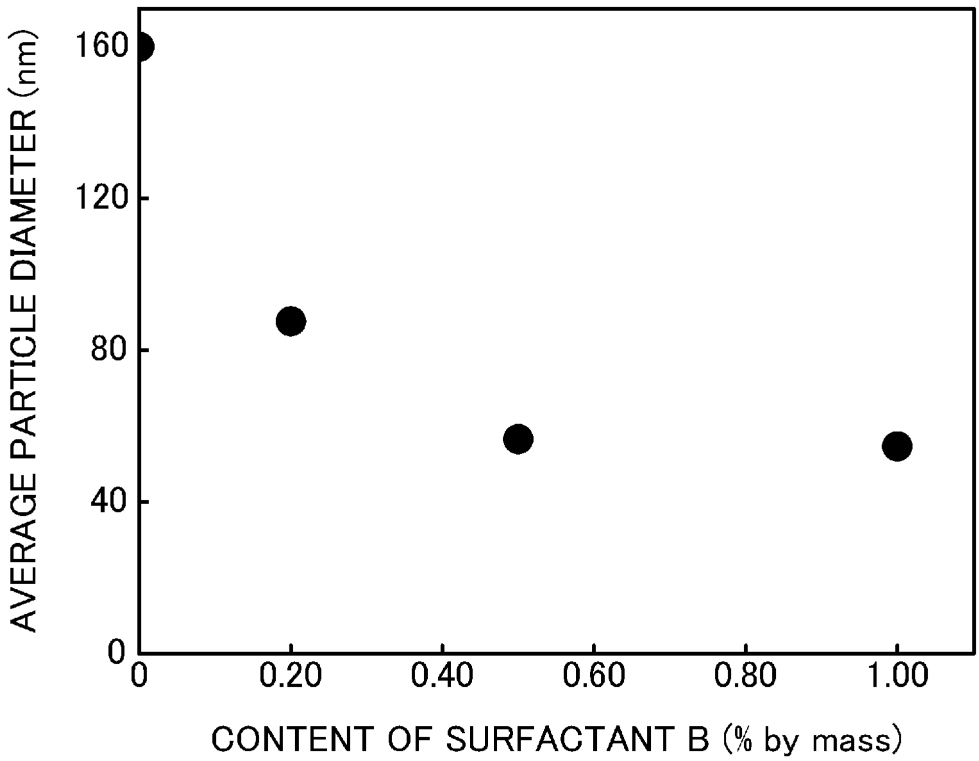
AVERAGE PARTICLE DIAMETER (nm)
160
120
80
40
0
0
0.20
0.40
0.60
0.80
1.00
CONTENT OF SURFACTANT B (% by mass)

FIG.7B

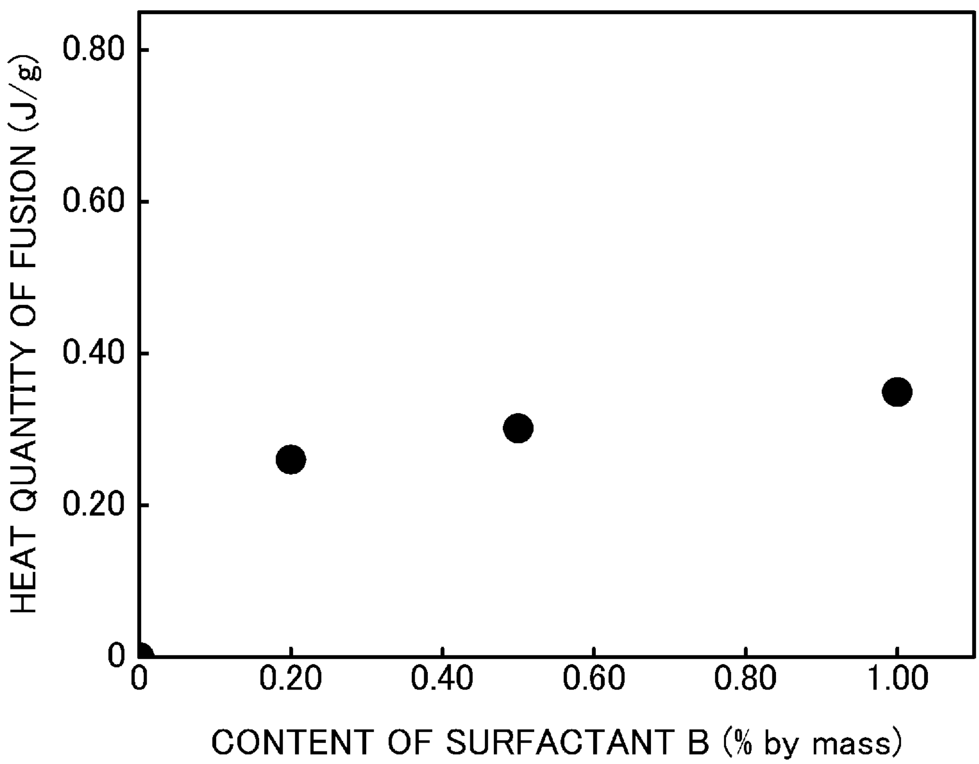
HEAT QUANTITY OF FUSION (J/g)
0.80
0.60
0.40
0.20
0
0
0.20
0.40
0.60
0.80
1.00
CONTENT OF SURFACTANT B (% by mass)

## INTERNATIONAL SEARCH REPORT

International application No.
PCT/JP2017/046172

A. CLASSIFICATION OF SUBJECT MATTER
Int.Cl. B01J13/00(2006.01)i, A61K8/04(2006.01)i, A61K8/63(2006.01)i, A61K8/68(2006.01)i

According to International Patent Classification (IPC) or to both national classification and IPC

B. FIELDS SEARCHED
Minimum documentation searched (classification system followed by classification symbols)
Intel. B01J13/00, A61K8/00-99, A61Q1/00-90/00, A61K9/00-72, A61K47/00-69, A61P1/00-43/00, B01F3/08

Documentation searched other than minimum documentation to the extent that such documents are included in the fields searched

| | |
|---|---|
| Published examined utility model applications of Japan | 1922-1996 |
| Published unexamined utility model applications of Japan | 1971-2018 |
| Registered utility model specifications of Japan | 1996-2018 |
| Published registered utility model applications of Japan | 1994-2018 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| X<br>Y | JP 2012-214470 A (KAO CORP.) 08 November 2012, claims, examples 3, 4, 6, 10 & US 2014/0018443 A1, claims, examples 3, 4, 6, 10 & WO 2012/133817 A1 & EP 2692334 A1 & CN 103458866 A | 13-16<br>1-12 |
| X<br>Y | JP 11-130651 A (KOSÉ CORPORATION) 18 May 1999, claims, example 8 (Family: none) | 13, 15<br>1-4, 6, 10, 12 |
| Y | JP 2007-8924 A (KAO CORP.) 18 January 2007, claims, paragraph [0045], examples (Family: none) | 1-12 |
| Y | JP 2011-20089 A (FUJIFILM CORP.) 03 February 2011, claims, paragraph [0089], examples & US 2011/0015416 A1, claims, paragraph [0094], examples | 1-12 |

☐ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 15 January 2018 (15.01.2018) | 23 January 2018 (23.01.2018) |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**INTERNATIONAL SEARCH REPORT**

International application No.
PCT/JP2017/046172

**Box No. II Observations where certain claims were found unsearchable (Continuation of item 2 of first sheet)**

This international search report has not been established in respect of certain claims under Article 17(2)(a) for the following reasons:

1. ☐ Claims Nos.:
because they relate to subject matter not required to be searched by this Authority, namely:

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:
See extra sheet

1. ☐ As all required additional search fees were timely paid by the applicant, this international search report covers all searchable claims.
2. ☒ As all searchable claims could be searched without effort justifying additional fees, this Authority did not invite payment of additional fees.
3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**

☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (January 2015)

INTERNATIONAL SEARCH REPORT

International application No.
PCT/JP2017/046172

Due to the reasons given below, this international application includes two inventions which do not satisfy the requirements for unity of invention

(Invention 1) Claims 1-12
(Invention 2) Claims 13-16

Claims 1-12 have the special technical feature of a "method of manufacturing a ceramide fine particle dispersion material, the method comprising a merging step of merging a first mixture of a ceramide and a liquid containing sterols, and a second mixture of a liquid containing water, while keeping each of the first mixture and the second mixture flowing; and a pore circulating step of circulating the fluid, obtained by merging the first mixture and the second mixture in the merging step, into holes having diameters of 0.03 mm to 20 mm inclusive, wherein, in the first mixture, the mass ratio of the content of a water-soluble organic solvent to the content of said ceramide is 0 to 30 inclusive".

Claims 13-16 have the technical feature of a "ceramide fine particle dispersion material" in common with claim 1. However, said technical feature does not make a contribution over the prior art in light of the disclosures of documents 1-4 below, and thus cannot be said to be a special technical feature. In addition, there are no other identical or corresponding special technical features between these inventions.

In addition, claims 13-16 are not dependent on claim 1. In addition, claims 13-16 are not substantially identical or equivalent to any of the claims classified as Invention 1.

Thus, claims 13-16 cannot be classified as Invention 1.

Document 1: JP 2012-214470 A (KAO CORP.) 08 December 2012
Document 2: JP 11-130651 A (KOSE CORP.) 18 May 1999
Document 3: JP 2007-8924 A (KAO CORP.) 18 January 2007
Document 4: TP 2011-20089 A (FUJIFILM CORP.) 03 February 2011

Form PCT/ISA/210 (extra sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2008037842 A
- JP 2015024404 A
- JP 2015174840 A
- JP 2013224314 A
- WO 2009145299 A
- JP 2014208626 A


### Non-patent literature cited in the description


- Nyuka Kayoka no Gijutsu. Kogaku Tosho (Kabu), 8-12
- **P. GUICHARDON ; L. FALK.** *Chemical Engineering Science,* 2000, vol. 55, 4233-4243